# EUROPEAN PATENT APPLICATION

(11) **EP 1 043 336 A2**
(43) Date of publication of application: **11.10.2000**
(21) Application number: 00302281.1
(22) Date of filing: 21.03.2000
(51) Int. Cl.: C07K 16/44, C07K 1/22, G01N 33/53, G01N 33/543

(54) **Stereospecific antibodies for imidazolinone herbicides**

(30) Priority: 29.03.1999 US 280182
(71) Applicant: The Regents of the University of California, Oakland, CA 94607-5200 (US); American Cyanamid Company, Parsippany, New Jersey 07054 (US)
(72) Inventor: Karu, Alexander Edwin, Kensington, California 94708 (US); Wong, Rosie Bick-Har, Piscataway, New Jersey 08854 (US); Chin, Tina Elain, Albany, California 94706 (US); Pont, Joseph Luke, Newtown, Pennsylvania 18940 (US)
(74) Representative: Browne, Robin Forsythe, Dr.

(57) **Abstract**

Provided are monoclonal antibodies and derivatives, including single-chain Fv antibodies, with chiral specificity for an enantiomer of an imidazolinone herbicide. Stereospecific monoclonal antibodies and derivatives in accordance with the present invention may be used in methods for the analysis, separation and purification of chiral isomers of imidazolinone herbicides, as well as in immunoaffinity chromatography separation columns for chiral isomers of imidazolinone herbicides, and other immunoaffinity assays.

## Description

### CROSS-REFERENCE TO RELATED APPLICATIONS

This application is a continuation-in-part of U.S. Patent Application No. 08/782,698 entitled IMIDAZOLINONE HAPTENS AND ANTIGENS AND ENZYME CONJUGATES PREPARED THEREFROM, filed January 16, 1997; which is a division of U.S. Patent Application No. 08/333,826 entitled IMIDAZOLIINONES, filed November 3, 1994 (now US Patent No. 5,693,658, issued December 2, 1997); each of which is incorporated herein by reference for all purposes.

### BACKGROUND OF THE INVENTION

This invention relates generally to imidazolinone herbicides, and in particular to monoclonal antibodies to imidazolinone herbicides. More specifically, the invention relates to monoclonal antibodies which exhibit chiral specificity to imidazolinone herbicides.

The imidazolinone family of herbicides are potent and selective inhibitors of acetohydroxyacid synthase (AHAS), a key enzyme that is a control point in the pathway of leucine, valine, and isoleucine biosynthesis in plants (Shaner, D. L., et al. 1984. Imidazolinones: potent inhibitors of acetohydroxyacid synthase. *Plant Physiology* 76: 545-546). This family of herbicides presently includes imazaquin, imazethapyr, imazapyr, imazamethabenz methyl, imazapic, and imazamox. These are the active ingredients of SCEPTER®, PURSUIT®, ARSENAL®, ASSERT®, CADRE®, and RAPTOR®, respectively, illustrated below in Table 1.

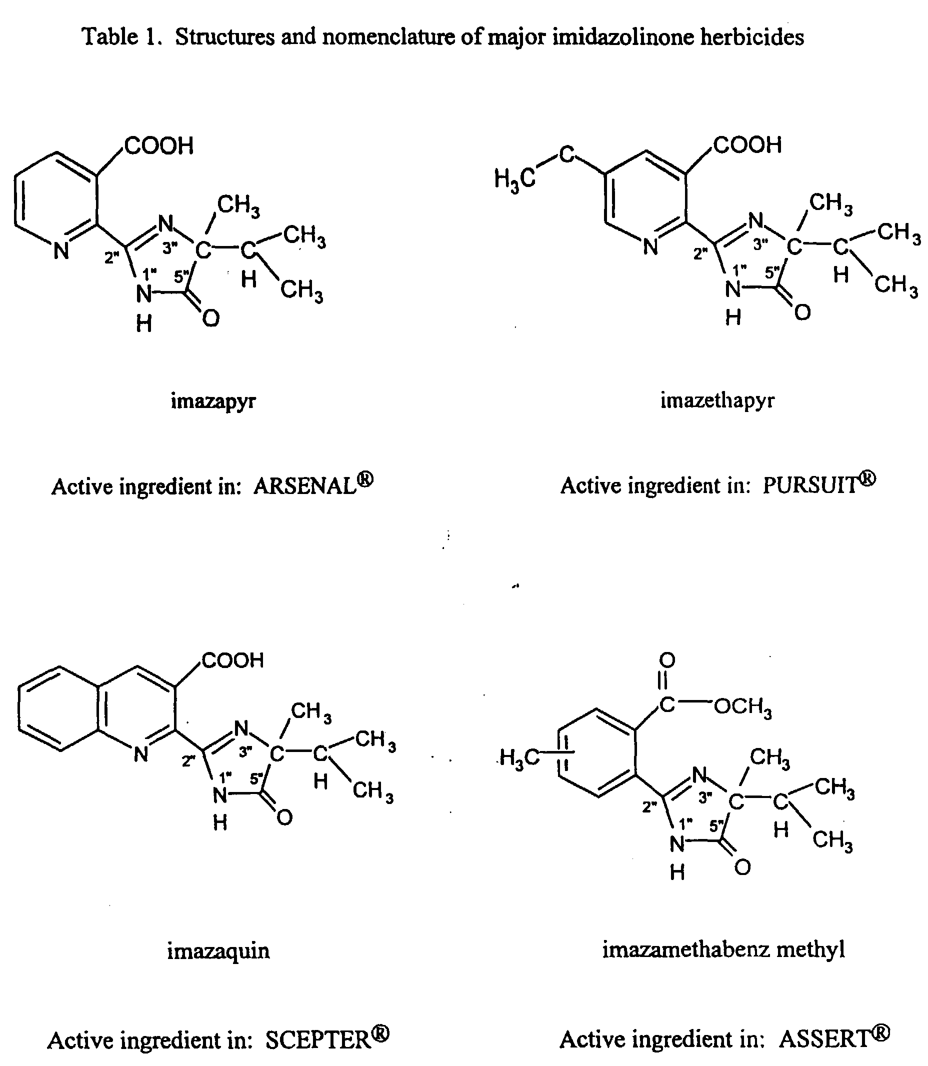

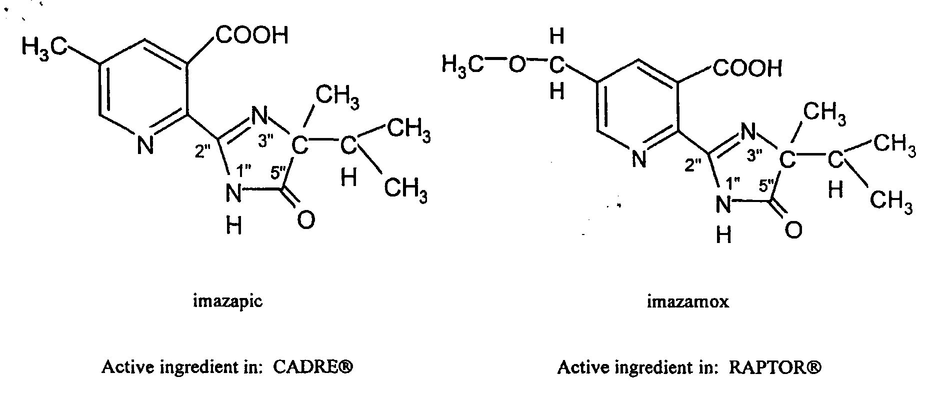

The mode of action of the six imidazolinones presently used in commercially important herbicide formulations, noted above, has been extensively investigated. The imidazoinone class is shown to bind in an uncompetitive manner to the AHAS-pyruvate complex (Shaner et al., 1984). A spectrophotometric assay for AHAS was used to demonstrate that there are at least two AHAS isozymes, designated I and II, that could be purified from Black Mexican Sweet corn (Singh, B. K.; Newhouse, K. E.; Stidham, M. A.; Shaner, D. L., Ed.; Acetohydroxyacid synthase-imidazolinone interaction. British Crop Protection Council: Farnham, Surrey, U.K., 1989. 87-95). AHAS II proved to be significantly more sensitive than AHAS I to inhibition by imazapyr. AHAS I is sensitive to inhibition by leucine and valine while AHAS II is not. Imidazolinones are taken into leaf cells and retained by an active transport and "ion-trap" effect, instead of by passive diffusion, as for atrazine or amitrole, or with a carrier, as for 2,4-D (Van Ellis, M. R; Shaner, D. L. Mechanism of cellular absorption of imidazolinones in soybean (*Glycine max*) leaf discs. *Pest Sci.* 1988, 23, 25-34).

The imidazolinones are considered to be "environmentally friendly," because they have very low toxicity toward mammals, birds and fish, which do not have the enzyme AHAS. None of the imidazolinones is genotoxic in any of the established *in vitro* and *in vivo* mutagenicity tests (Gagne, J. A., et al. 1991. Toxicology of the imidazolinone herbicides. In: *The Imidazolinone Herbicides*, eds. DL Shaner, SL O'Connor, pp. 179-182. Boca Raton: CRC Press). Imidazolinones are potent when applied, but they have very favorable biodegradation and dissipation properties. The properties of these compounds with respect to leachability, adherence to soil substances, retention after rainfall, photolysis, etc., are superior to those of many presently used herbicides with similar properties.

Some imidazolinones are broad-spectrum while others act selectively on weeds in the presence of tolerant crops such as soybeans, corn, wheat, barley, rye, and peanuts. Selectivity depends predominantly on differences in imidazolinone metabolism by different plant species. Plants tolerant to imazamethabenz-methyl (ASSERT®) convert it to a conjugated metabolite. Species that tolerate imazapyr (ARSENAL®) and imazaquin (SCEPTER®) metabolize these to nontoxic forms, while tolerance to imazethapyr (PURSUIT®) appears due to differential rates of metabolism or conjugation. (Shaner, D. L.; Mallipudi, N. M. Mechanisms of selectivity of the imidazolinones. In *The Imidazolinone Herbicides*; Shaner, D. L., O'Connor, S. L., Eds.; CRC Press: Boca Raton, 1991).

Imidazolinones are slowly broken down in soil. Residue monitoring is important for effective weed control without damage to crops or emergence of resistant weeds, as well as for compliance with good health, safety, and environmental practices and associated regulations. Several methods have been reported for recovery and analysis of imidazolinone residues from soil, water, forestry samples, sunflower seed, straw, leguminous vegetables, urine, kidney homogenates, etc. (Devine, J. M. Residue Analysis. In *The Imidazolinone Herbicides;* Shaner, D. L., O'Connor, S. L., Eds.; CRC Press: Boca Raton, FL, 1991; 173-178; Wong, R B.; Pont, J.; Johnson, D.; Zulalian, J.; Chin, T.; Karu, A. E. Immunoaffinity chromatography applications in pesticide metabolism and residue analyses. In *Immunoanalysis of Agrochemicals: Emerging Technologies;* Nelson, J., Karu, A. E., Wong, R., Eds.; American Chemical Society: Washington D.C., 1995; 235-247). Chemical extraction protocols for these diverse matrices are lengthy and the recovery efficiencies vary. U.S. Patent Application Serial No. 08/782,698, which is incorporated herein by reference for all purposes, discloses a panel of monoclonal antibodies (MAbs) that detect imazethapyr and other imidazolinone herbicides in the low ppb to ppm range, which may be useful for immunoaffinity chromatography for residue analysis and other monitoring activities.

In addition to these residue analysis and other monitoring activities, it would also be desirable to minimize the amount of material initially applied in herbicidal formulations in order to improve cost-effectiveness and reduce herbicide residue in the environment. The imidazole ring in imidazolinone herbicide compounds has a chiral carbon to which the methyl and isopropyl groups are attached. The *R* isomer of imazethapyr and other pyridine imidazolinones has been found to be tenfold more inhibitory to AHAS than the *S* isomer (Stidham, M. and Singh, B. Imidazolinone-acetohydroxy acid synthase interactions. In *The Imidazolinone Herbicides;* Shaner, D. L., O'Connor, S. L., Eds.; CRC Press: Boca Raton, FL, 1991; 71-90).

Accordingly, a chemical binding agent with chiral specificity for one of the enantiomers of an imidazolinone herbicide active agent would be useful for analysis and purification of the diastereomers from mixtures of these compounds prior to their formulation and application as herbicides.

### SUMMARY OF THE INVENTION

To achieve the foregoing, the present invention provides monoclonal antibodies and derivatives with chiral specificity for an enantiomer of an imidazolinone herbicide. In preferred embodiments, the invention provides monoclonal antibodies (MAbs) and a single-chain Fv antibody (scFv) derived from a MAb by molecular cloning (recombinant DNA) methods. The MAbs and scFv have chiral specificity, for example, a binding preference for the *S*-enantiomers of imidazolinone herbicides. These stereospecific antibodies and their derivatives may be used in virtually any immunoassay format or antibody-based sensor device for detecting, identifying, and quantifying imidazolinones, and in immunoaffinity methods for concentration, cleanup, and separation of chiral isomers of the imidazolinones. Potential applications include analyzing fate and transport of the herbicides to meet pesticide registration requirements, monitoring the herbicide synthesis and formulation processes, and screening of potential new herbicides of this chemical class.

In one aspect, the present invention provides a monoclonal antibodies for an imidazolinone herbicide which exhibits chiral specificity for an enantiomer of the imidazolinone herbicide.

In another aspect, the invention provides immunoaffinity assays and separation methods for chiral isomers of an imidazolinone herbicide. The methods involve contacting a sample including one or more imidazolinones with a monoclonal antibody which exhibits chiral specificity for an enantiomer of an imidazolinone herbicide.

In another aspect, the invention provides apparatuses and column for conducting an immunoaffinity assays and separations for chiral isomers of an imidazolinone herbicide. The apparatuses and cloumns include a monoclonal antibody which exhibits chiral specificity for an enantiomer of an imidazolinone herbicide, immobilized on a solid material.

Other aspects of the present invention provide a recombinant single-chain Fv antibody for an imidazolinone herbicide which exhibits chiral specificity for an enantiomer of the imidazolinone herbicide, and immunoaffinity assays, separation methods, and apparatuses incorporating such antibodies.

These and other features and advantages of the present invention will be presented in more detail in the following specification of the invention and the accompanying figures which illustrate by way of example the principles of the invention.

### BRIEF DESCRIPTION OF THE DRAWINGS

Figure 1 depicts a scheme for chemical synthesis of haptens with spacer arms attached on the pyridine ring, that served as mimics of imazethapyr for evoking, selecting, and using antibodies in accordance with the present invention.
Figures 2 and 3 show steps in a chemical synthesis of haptens with spacer arms attached on the imidazole ring, that served as mimics of imazethapyr for evoking, selecting, and using antibodies in accordance with the present invention.
Figure 4 shows haptens used as competitors in direct and indirect enzyme immunoassays (EIAs) with MAbs and scFv antibody in accordance with the present invention.
Figure 5 is a table comparing the sensitivities of indirect EIAs with the five MAbs and hapten conjugates described in this disclosure.
Figures 6A and 6B show the effects of different concentrations of methanol and acetonitrile, respectively, on binding of each of five MAbs to screening conjugates in indirect EIAs.
Figure 7 summarizes the sensitivity of indirect EIAs for chiral isomers of the five major imidazolinone herbicides, using each of the five MAbs described in this disclosure.
Figure 8 presents schematic diagrams of the protein structures of MAb, recombinant Fab, and scFv antibodies.
Figure 9 is a flow chart of the major steps in derivation of the scFv from MAb 3A5 in accordance with the present invention.
Figure 10 shows the haptens used to select and work with scFv 3A5. For consistency, the structures are numbered the same as they were in Figures 1-4.
Figure 11 is the genetic map of the phagemid DNA vector constructed from pCANTAB 5E to clone and express scFv 3A5.
Figures 12A and 12B depict bar graphs showing that the amount of inducing agent (IPTG) for optimum expression of functional scFv 3A5 is about 0.05-0.1 mM.
Figure 13 is a summary of the selectivity of scFv 3A5 for imidazolinone chiral isomers in an indirect EIA.
Figure 14 tabulates the selectivity of scFv 3A5 for imidazolinone chiral isomers in a direct EIA.
Figure 15A and B show relative binding of known amounts of MAb 3A5 and scFv 3A5 to the 16-BSA and 17-OA haptens, respectively, as a comparison of affinities.
Figure 16 compares the stabilities of MAb and scFv after incubation in methanol or acetonitrile, which are solvents that may be used for imidazolinone residue analysis.
Figure 17 presents the entire amino acid (SEQ.ID NO. 3) and nucleotide (SEQ.ID NO. 4) sequence of the scFv 3A5, including the linker region and the mini-FLAG and Strep-tag affinity tag sequences that were introduced by mutagenesis at the N- and C-termini, respectively.
Figure 18 depicts the N-terminal amino acid sequences of the V_{H} and V_{L} domains of the 3A5 MAb (SEQ.ID NOS. 1 and 2, respectively) and scFv (SEQ.ID NOS. 5 and 6, respectively).

### DETAILED DESCRIPTION OF PREFERRED EMBODIMENTS

The present invention will now be described with reference to several preferred embodiments. Important properties and characteristics of the preferred embodiments are illustrated in the structures in the text and in the accompanying drawings. While the invention will be described in conjunction with these preferred embodiments, it should be understood that the invention is not intended to be limited to these preferred embodiments. On the contrary, it is intended to cover alternatives, modifications, and equivalents as may be included within the spirit and scope of the invention as defined by the appended claims. In the following description, numerous specific details are set forth in order to provide a thorough understanding of the present invention. The present invention may be practiced without some or all of these specific details. In other instances, well known process operations have not been described in detail in order not to unnecessarily obscure the present invention.

The present invention provides monoclonal antibodies and derivatives with chiral specificity for an enantiomer of an imidazolinone herbicide. Stereospecific monoclonal antibodies and derivatives in accordance with the present invention may be used in methods for the detection and analysis of imidazolinone chiral isomers, as well as in immunoaffinity chromatography separation columns for chiral isomers of imidazolinone herbicides.

Hybridomas producing stereospecific MAbs in accordance with the present invention were derived using haptens and hapten conjugates described in parent patent application Serial No. 08/782,698 and grandparent patent application Serial no. 08/333,826 (U.S. Patent no. 5,693,658), the disclosures of which have previously been incorporated by reference herein for all purposes.

In one preferred embodiment of the present invention, MAbs with chiral specificity may be evoked by some of the disclosed imidazolinone haptens, and identified in competition immunoassays using other disclosed haptens as the competitor. Other methods, such as instrumental analysis (for example, nuclear magnetic resonance) may also be used to reveal chiral specificity. Exemplary details of various aspects of how MAbs in accordance with the present invention may be produced, identified, and characterized are provided below.

In a second embodiment, the variable regions that are responsible for the binding properties of chirally specific MAbs may be isolated by molecular cloning of the coding sequences from the hybridoma cells that express the MAbs. Established molecular biological techniques may then be used to express the chirally specific scFvs in bacteria, other microorganisms, or higher plants. Exemplary details of the preparation, expression, and characterization of scFv antibodies stereospecific for imidazolinones in accordance with the present invention.

The following is a list of generic names and trade names, together with their corresponding IUPAC names and CAS Registry numbers, which may be a useful reference to assist in understanding the following disclosure of the present invention: Imazethapyr (PURSUIT®); 5-ethyl-2-(4-isopropyl-4-methyl-5-oxo-2-imidazolin-2-yl) nicotinic acid, 81335-77-5. Imazapyr (ARSENAL®) 2-(4-isopropyl-4-methyl-5-oxo-2-imidazolin-2-yl) nicotinic acid, 81510-83-0. Imazamethabenz-methyl (ASSERT®); a mixture of methyl 6-(4-isopropyl-4-methyl-5-oxo-2-imidazolin-2-yl) *m*-toluate and methyl 2-(4-isopropyl-4-methyl-5-oxo-2-imidazolin-2-yl) *p*-toluate, 81405-85-8. Imazaquin (SCEPTER®); 2-(4-isopropyl-4-methyl-5-oxo-2-imidazolin-2-yl)-3-quinoline carboxylic acid, 81335-47-9, imazapic (CADRE®), ®); 5-methyl-2-(4-isopropyl-4-methyl-5-oxo-2-imidazolin-2-yl) nicotinic acid, 81334-60-3, imazamox (RAPTOR®), 5-methoxyl-2-(4-isopropyl-4-methyl-5-oxo-2-imidazolin-2-yl) nicotinic acid, 114311-32-9. Acifluorfen (BLAZER®, TACKLE®); 5-[2-chloro-4-(trifluoromethyl)phenoxy]2-nitrobenzoate, 50594-66-6. Alachlor, (LASSO®) 2-chloro-2'-6'-diethyl-N-(methoxymethyl)-acetanilide, 15972-60-8. Atrazine, 1912-24-9. Primisulfuron (BEACON®), 3-[4,6-bis(difluoromethoxy)-pyrimidin-2-yl]-1-(2-methoxycarbonyl-phenylsulfonyl)-urea, 86209-51-0. EPTC (EPTAM®), S-ethyl dipropylthiocarbamate, 759-94-4. Glyphosate (ROUNDUP®), N-(phosphono-methyl) glycine isopropylamine salt, 1071-83-6. Metolachlor, 2-chloro-N-(2-ethyl-6-methylphenyl)-N-(2-methoxy-1-methylethyl) acetamide, 51218-45-2. Molinate (ORDRAM®), S-ethyl-hexahydro-1-H-azepine-1-carbothioate, 2212-67-1. Oxyfluorfen (GOAL®), 2-chloro-1-(3-ethoxy-4-nitrophenoxy)-4-(trifluoromethyl) benzene, 42874-03-3. Paraquat, 1,1'-dimethyl-4,4'-bipyridinium dichloride, 1910-42-5. Pendimethalin (PROWL®), N-(1-ethylpropyl)-3,4-dimethyl-2,6-dinitrobenzenamine, 040487-42-1. Phenmedipham, 3-methoxycarbonylaminophenyl-N(3'-methylphenyl)carbamate, 13684-63-4. Sethoxydim (POAST), 2[1-ethoxyimino)butyl]-5-[2-ethylthio)propyl-3-hydroxy-2-cyclohexen-1-one, 74051-80-2. Thiobencarb (BOLERO®), S-[(4-chlorophenyl)methyl]diethylcarbamothioate. Chlorimuron ethyl (CLASSIC®), ethyl 2-[[[[(4-chloro-6-methoxypyrimidin 2-yl)amino]carbonyl]amino] sulfonyl]benzoate, 90982-32-4. Chlorsulfuron (GLEAN®), 2-chloro-N[(4-methoxy-6-methyl-1,3,5 triazin-2-yl)aminocarbonyl]benzenesulfonamide, 64902-72-3.

In addition, the following abbreviations are used in the following disclosure: V_{H}, V_{L} , the heavy and light chain variable domains of an antibody; pfu, phage plaque forming units determined as infective centers; cfu, colony forming units, determined in an assay for drug resistance. Amino acids in peptide linkage are referred to by the first three letters, e.g., Phe = phenylalanine, except that Gln = glutamine and Asn = asparagine.

The following procedures and results provide details of the chemical syntheses and methods used to produce stereospecific MAbs in accordance with the present invention. However, these procedures and results should be considered as exemplary of the invention only, and the invention is not limited by the details thereof.

### I. Development of Imidazolinone-Specific Monoclonal Antibodies and Immunoassays

### Procedure 1: Synthesis of Haptens

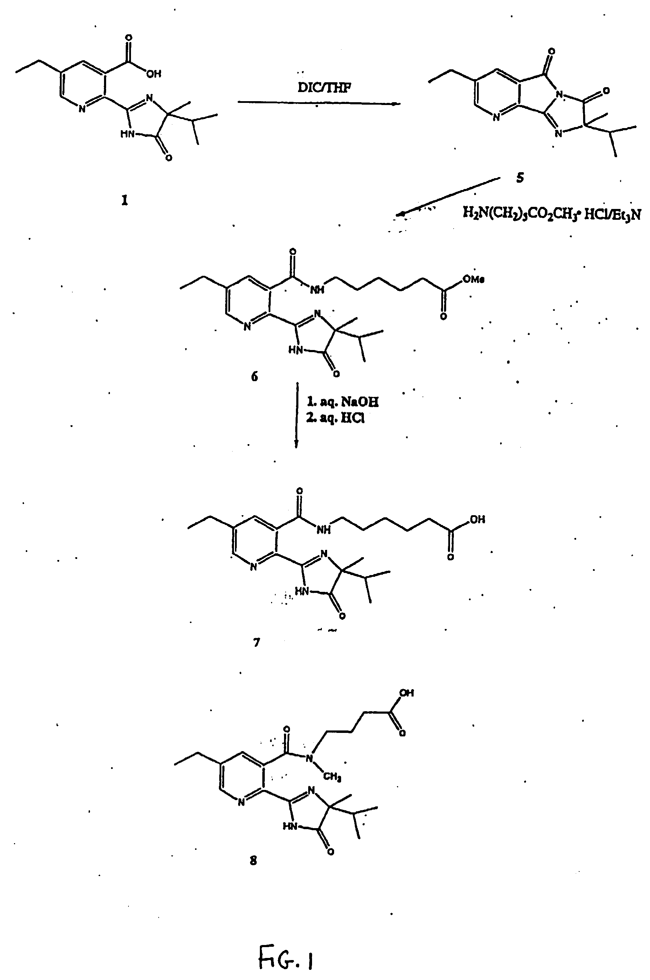

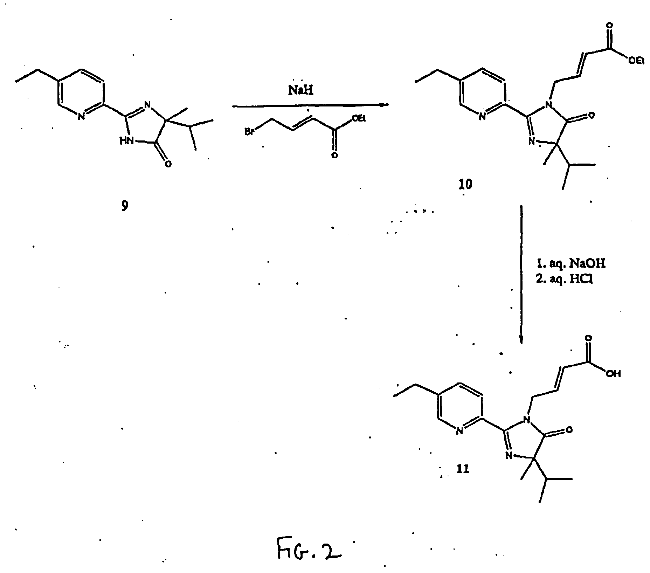

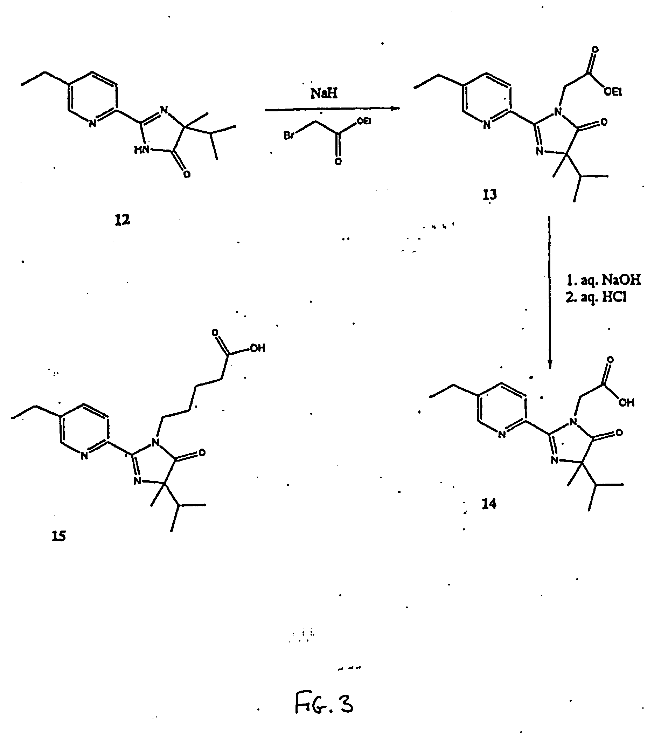

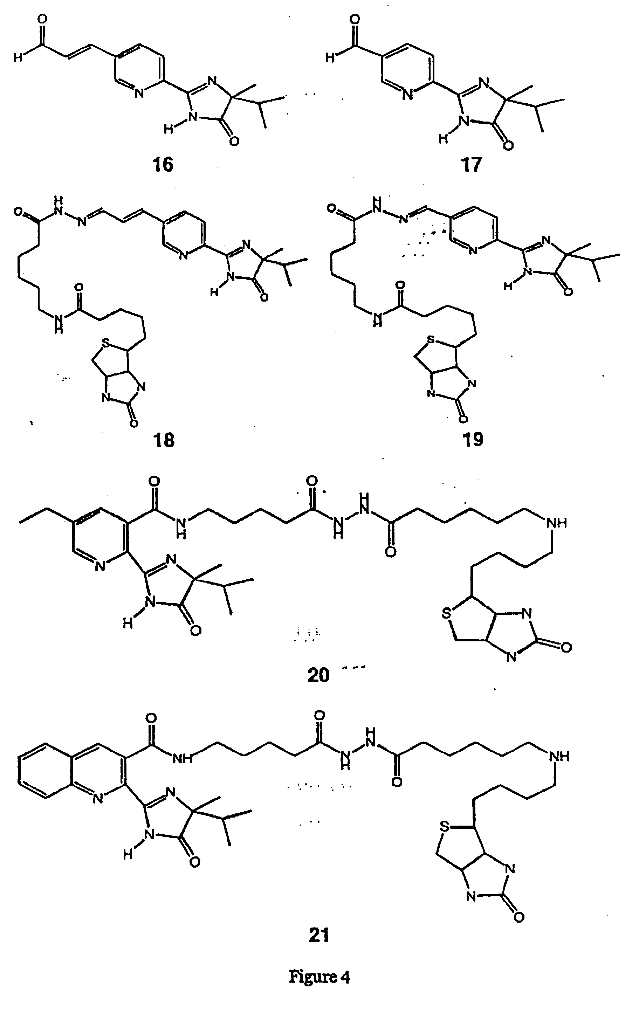

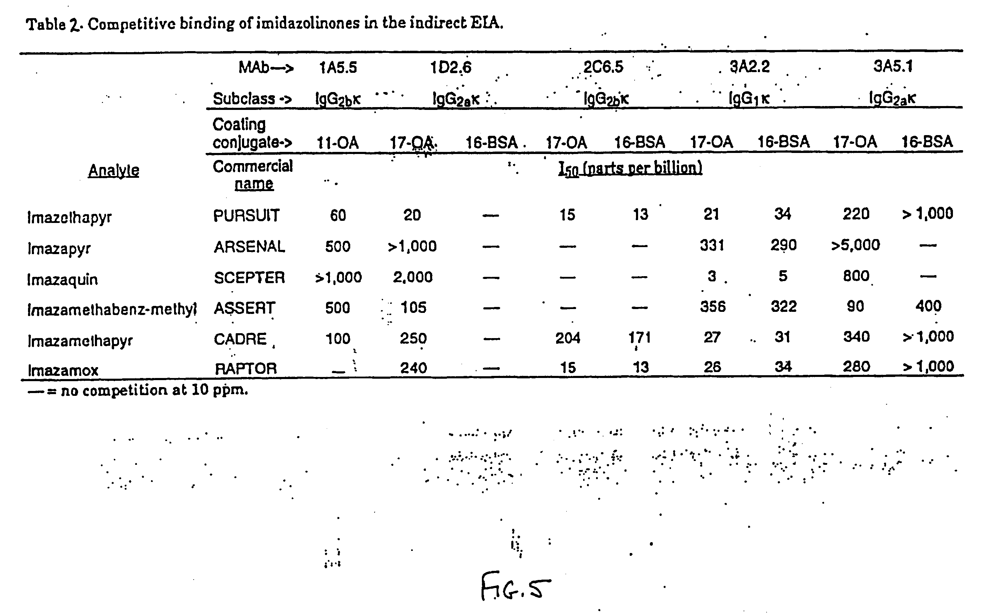

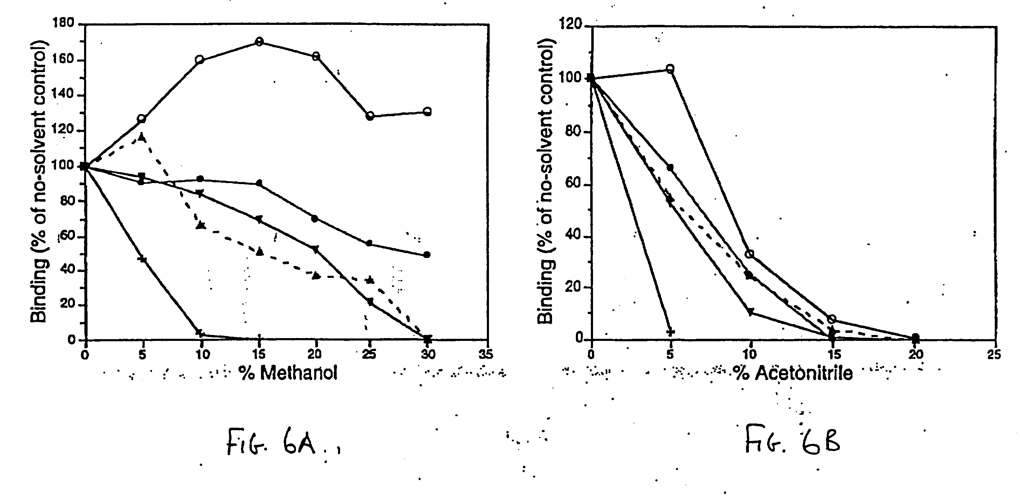

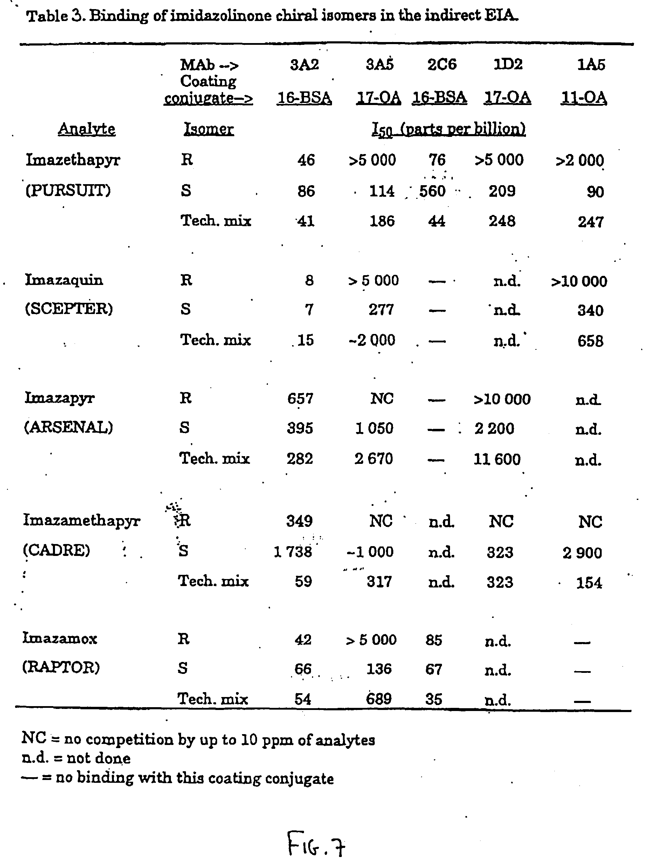

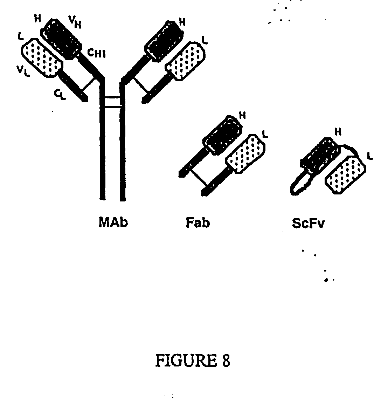

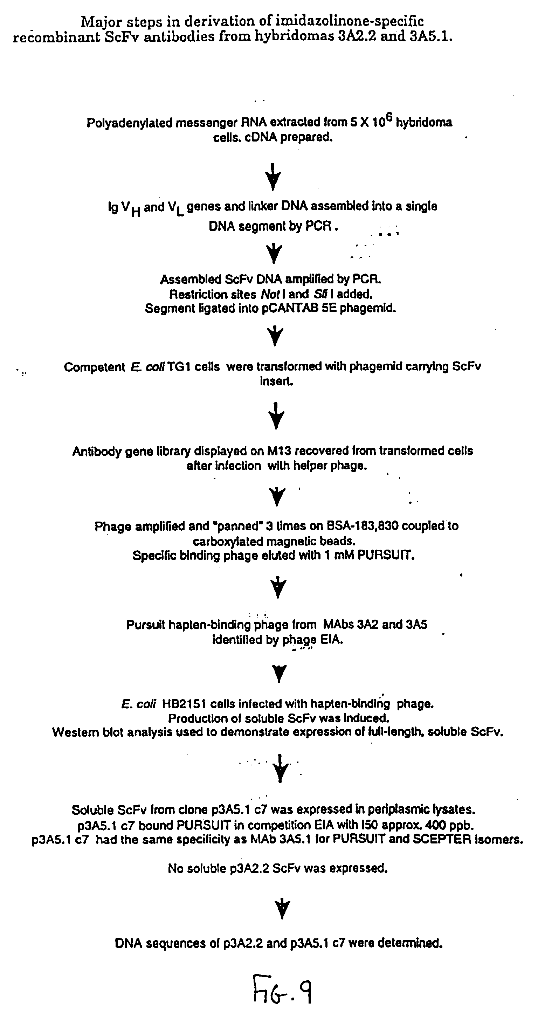

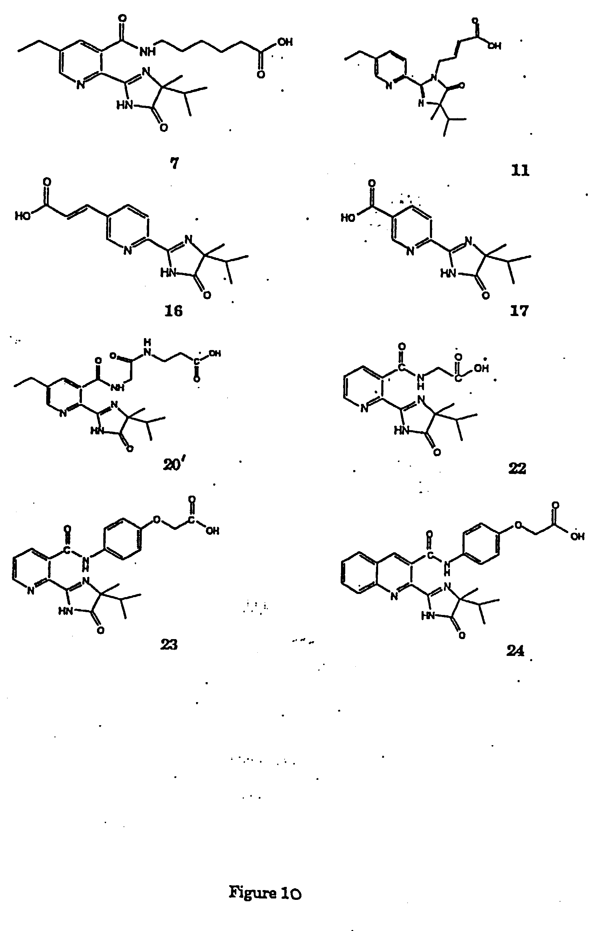

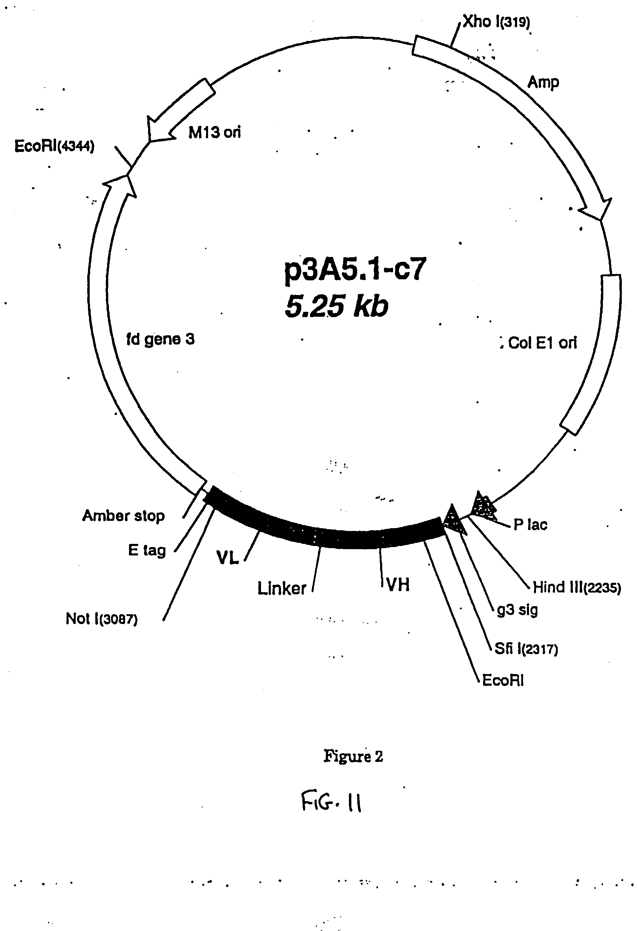

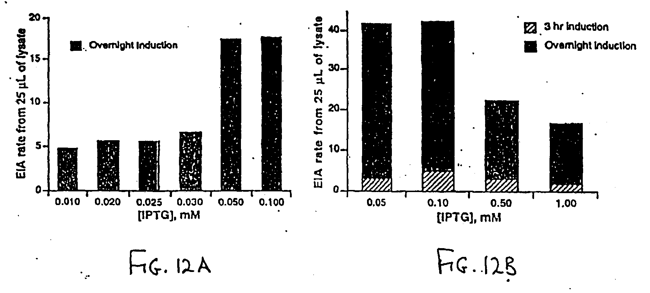

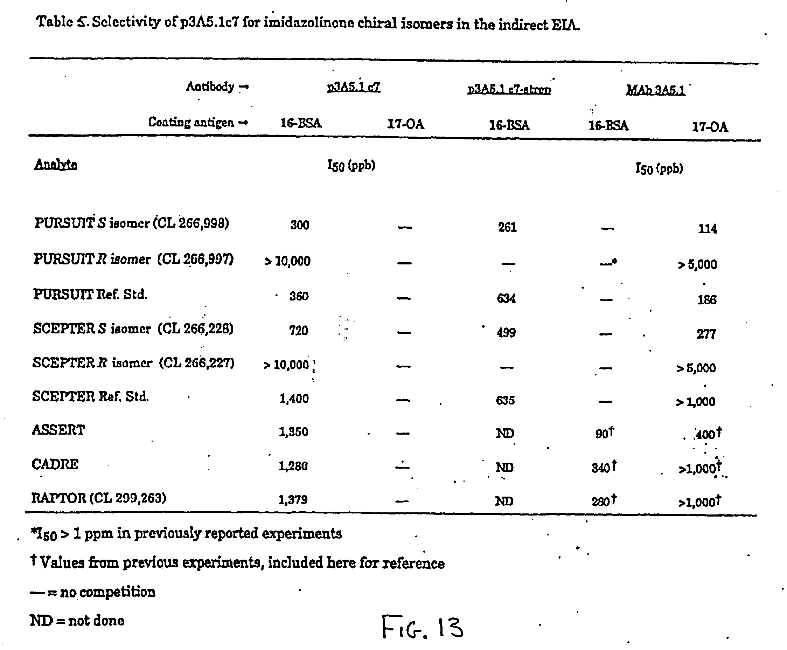

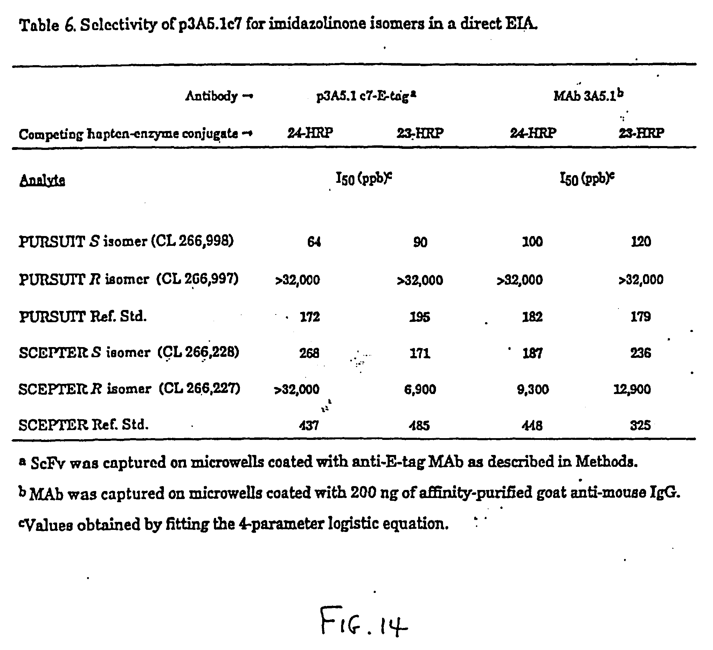

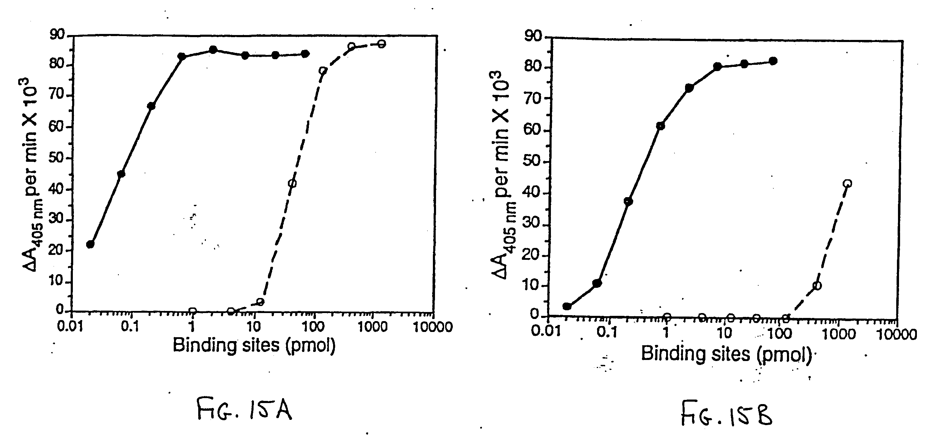

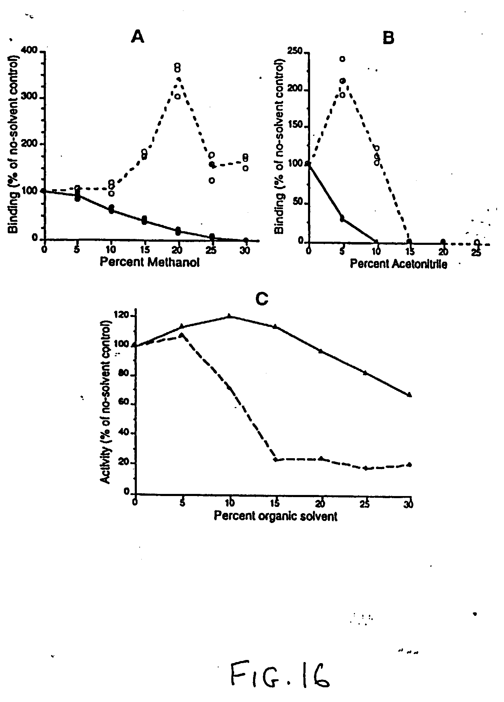

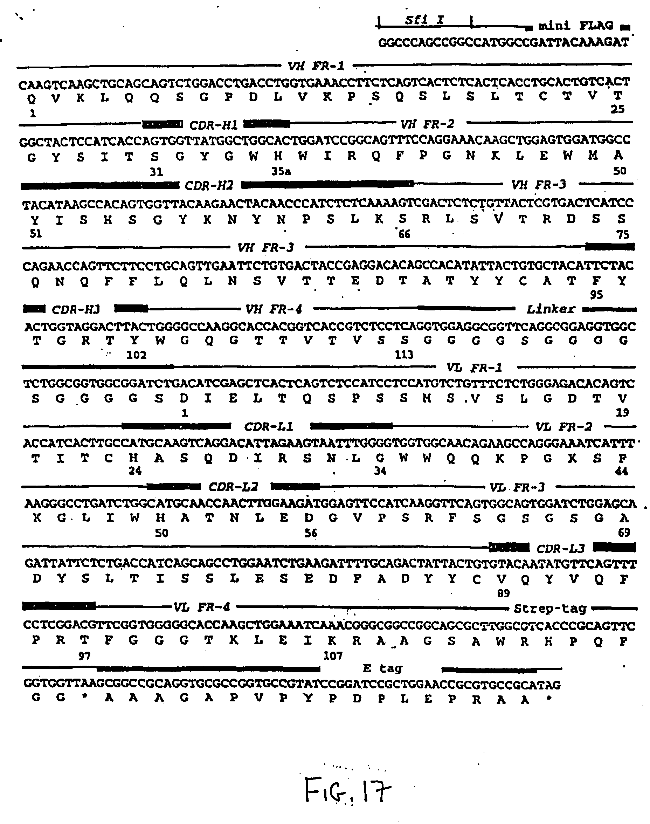

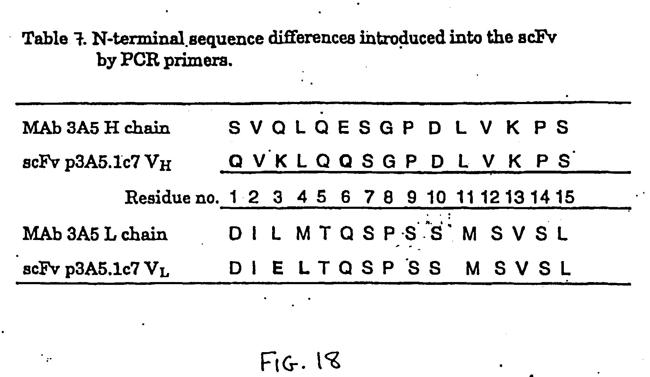

Haptens with the spacer attached at the carboxylic acid on the pyridine ring were synthesized in two steps from imazethapyr **1** as shown in Figure 1 (Los, M. Imidazoisoindolenediones and use thereof as herbicidal agents. U. S. *Patent 4,017,510,* 1977; Los, M. Method of preparing imidazoisoindolenediones. *U. S. Patent 4,125,727,* 1978):

*Methyl 6-[5-ethyl-2-(4-isopropyl-4-methyl-5-oxo-2-imidazolin-2-yl) nicotinamido] hexanoate* **6**: A stirred solution of 7-ethyl-2-isopropyl-2-methyl-5H-imidazo [1',2':1,2]pyrrolo[3,4-b]pyridine-3(2H),5-dione **5** (7.25 g, 26.75 mmol) in anhydrous N,N-dimethylformamide (DMF) was treated successively with methyl 6-aminocaproate hydrochloride (5.10 g, 28.1 mmol) and triethylamine (3.00 g, 29.6 mmol). The resulting mixture was stirred at room temperature under nitrogen for 20.5 hours then partitioned between water and ethyl acetate. The layers were separated, and the organic layer was washed with water, dried over magnesium sulfate and vacuum filtered through diatomaceous earth. The filtrate was concentrated *in vacuo* to yield a white solid, which was triturated with 1:1 ether:petroleum ether to give compound **6**(7.40 g, 69%, mp 101-103 °C). ¹H NMR (CDCl₃, 300 MHz) d 11.0 (bs, 1H); 9.1 (bs, 1H); 8.6 (s, 1H); 8.5 (s, 1H); 3.7 (s, 3H); 3.6-3.3 (m, 2H); 2.8 (q, J = 7.4 Hz, 2H); 2.3 (t, J = 7.4 Hz, 2H); 2.2-2.0 (m, 1H); 1.8-1.6 (m, 4H); 1.5-1.4 (m, 5H); 1.3 (t, J = 7.7 Hz, 3H); 1.1 (d, J = 6.9 Hz, 3H); 0.9 (d, J = 6.9 Hz, 3H).

*6-[5-ethyl-2-(4-isopropyl-4-methyl-5-oxo-2-imidazolin-2-yl)nicotinamido] hexanoic acid* **7**: A stirred solution of methyl 6-[5-ethyl-2-(4-isopropyl-4-methyl-5-oxo-2-imidazolin-2-yl)nicotinamido] hexanoate **6** (5.14 g, 12.35 mmol) in tetrahydrofuran was treated with 2N NaOH (15 mL). The resulting mixture was stirred at room temperature for 24 h, and then concentrated *in vacuo* to obtain a residue. The residue was cooled with an ice bath and acidified with concentrated HCl. The resulting precipitate was isolated by vacuum filtration and washed with water to give a white solid product, **7** (mp 76-81°C). ¹H NMR (CDCl₃, 300 MHz) d 10.9 (bs, 1H); 8.5 (s, 1H); 3.6-3.4 (m, 2H); 2.8 (q, J = 7.7 Hz, 2H); 2.4 (t, J = 7.4 Hz, 2H): 2.2-2.0 (m, 1H): 1.7-1.6 (m, 4H); 1.5-1.4 (m, 1H); 1.4 (s, 3H); 1.3 (t, J 7.7 Hz, 3 H); 1.1 (d, J = 6.0 Hz, 3 H); 0.9 (d, J = 6.0 Hz, 3H).

*4-[5-ethyl-2-(4-isopropyl-4-methyl-5-oxo-2-imidazolin-2-yl)-N-methylnicotinamido]butyric acid* **8**. A scheme similar to that in Figure 1 was used to prepare compound **8**. A stirred solution of 7-ethyl-2-isopropyl-2-methyl-5H-imidazo[1',2':1,2]pyrrolo[3,4-b]pyridine-3 (2H), 5-dione **5** (5.47 g, 20.19 mmol) in anhydrous DMF was treated successively with 4-(methylamino)butyric acid hydrochloride (3.29 g., 21.2 mmol) and triethylamine (4.29 g., 42.39 mmol). The resulting mixture was stirred at room temperature under nitrogen for 2.5 hours, then diluted with water, acidified with concentrated hydrochloric acid, and extracted with ether. The organic extract was dried over magnesium sulfate, vacuum filtered through diatoinaceous earth, and concentrated *in vacuo* to give compound **8** as a white solid, mp 42-49°C (dec). ¹H NMR (CDCl₃, 300 MHz) d 8.5 (d, J = 2.2 Hz, 1H); 7.5 (d, J = 1.9 Hz, 1H); 3.6-3.2 (m, 2H); 3.1 (s, 1.5H); 2.8 (s, 1.5H); 2.8-2.7 (m, 2H); 2.6-1.8 (m,4H); 1.4-1.2 (m, 7H); 1.1 (d, J = 6.9 Hz, 3H); 0.9 (d, J = 6.9 Hz, 3H).

Haptens with the spacer arm on the imidazole ring were prepared as shown in Figures 2 and 3:

*Ethyl 2-(5-ethyl-2-pyridyl)-4-isopropyl-4-methyl-5-oxo-2-imidazoline-1-crotonate* **11**. A stirred suspension of sodium hydride (1.6 g, 53 mmol, 80% oil suspension) in dry THF was treated with a solution of 2-(5-ethyl-2-pyridyl)-4-isopropyl-4-methyl-5-oxo-2-imidazoline **9** (10.24 g, 41.8 mmol) in dry THF by dropwise addition. After addition was complete and the effervescence subsided, ethyl 4-bromocrotonate (12.9 g, 50.1 mmol, 75% technical grade) was added immediately. The resulting mixture was stirred for 19 hours at room temperature under nitrogen. The reaction mixture was then partitioned between water and ether. The organic layer was recovered and dried over magnesium sulfate, decolorized with activated charcoal, vacuum filtered through diatomaceous earth, and concentrated *in vacuo* to a dark brown oil. The oil was purified by flash chromatography (2:1 hexanes/ethyl acetate eluent), to give compound **10** as a pale orange oil (3.78 g, 25%). ¹H NMR (CDCl₃, 300 MHz) d 8.5 (d, J = 2.2 Hz, 1H); 8.2 (bs, 1H); 7.6 (m, 1H); 6.8 (m, 1H); 5.8 (d, J = 15.9 Hz; 1H); 4.9 (dd, J₁ = 5.2 Hz, J₂ = 1.4 Hz, 2H); 4.1 (q, J = 7.1 Hz, 2H); 2.7 (q, J = 7.4 Hz, 2H); 2.2 (m, 1H); 1.4 (s, 3H); 1.3-1.2 (m, 6H); 1.1 (d, J = 6.9 Hz, 3H); 0.9 (d, J = 6.9 Hz, 3H).

Compound **10** (1.16 g, 3.25 mmol) in 1:1:THF:H₂O was treated with a single addition of sodium hydroxide (0.45 g, 11.3 mmol). The resulting mixture was stirred at reflux for 1.5 hours, then at room temperature for 16.5 hours. The mixture was subsequently diluted with water and washed with ether. The aqueous layer was acidified with 1N hydrochloric acid (15 mL), and extracted with ether. The organic phase was dried over sodium sulfate, decolorized with charcoal, and vacuum filtered through diatomaceous earth. The filtrate was concentrated *in vacuo* to give 2-(5-ethyl-2-pyridyl)-4-isopropyl-4-methyl-5-oxo-2-imidazoline-1-crotonic acid **11**, as a yellow solid, mp 53-58 °C. ¹H NMR (CDCl₃, 300 MHz) d 8.4 (d, J = 1.7 Hz, 1H); 8.0 (d, J = 7.9 Hz, 1H); 7.6 (dd, J₁ = 8.2 Hz, J₂ = 2.1 Hz; 1H); 6.7 (m, 1H); 5.6 (d, J = 15.7 Hz, 1H); 4.8 (dd, J₁ = 5.3 Hz, J₂ = 1.6 Hz, 2H); 2.6 (q, J = 7.4 Hz, 2H); 2.1 (m, 1H); 1.3 (s, 3H); 1.2 (m, 3H); 1.0 (d, J = 6.8 Hz, 3H); 0.8 (d, J = 6.8 Hz, 3H). Compound **11** slowly decomposes on standing.

*2-(5-ethyl-2-pyridyl)-4-isopropyl-4-methyl-5-oxo-2-imidozoline-1-acetic acid* **14.** As shown in Figure 3, a stirred mixture of sodium hydride (0.45 g, 15 mmol, 80% oil dispersion) in anhydrous tetrahydrofuran (THF) was treated with a solution of 2-(5-ethyl-2-pyridyl)-4-isopropyl-4-methyl-5-oxo-2-imidazoline **12** (12, 3.3 g, 13.47 mmol) in anhydrous THF, dropwise over 40 min. After this addition was complete and the effervescence subsided, ethyl bromoacetate (2.30 g, 13.5 mmol) was added. The reaction was allowed to stir for 24 hours at room temperature under nitrogen, and the mixture was then partitioned between water and ether. The organic layer was recovered, dried over magnesium sulfate, vacuum filtered through diatomaceous earth, and concentrated *in vacuo*. The residue was purified by flash chromatography (2:1 hexanes/ethyl acetate eluent) to give product **13** as a pale yellow oil. ¹H NMR (CDCl₃, 300 MHz) d 8.4 (s, 1H); 8.1, d, J = 8.0 Hz, 1H); 7.7-7.6 (m, 1H); 4.8 (s, 2H); 4.0 (q, J = 7.1 Hz, 2H); 2.7 (q, J = 7.7 Hz, 2H); 2.2-2.1 (m, 1H); 1.4 (s, 3H); 1.3 (t, J = 7.7 Hz, 3H); 1.2 (t, J = 7.1 Hz, 3H); 1.1 (d, J = 6.9 Hz, 3H); 0.9 (d,J = 6.9 Hz, 3H).

Compound **13** (2.12 g, 6.41 mmol) in THF was treated with 2N sodium hydroxide (8 mL), and the resulting mixture was stirred at room temperature for 16 h. It was then concentrated *in vacuo* to obtain a residue. The residue was acidified with concentrated hydrochloric acid and then extracted with ether. The organic extract was dried over magnesium sulfate, vacuum filtered through diatomaceous earth, and concentrated *in vacuo* to give 2-(5-ethyl-2-pyridyl)-4-isopropyl-4-methyl-5-oxo-2-imidazoline-1-acetic acid **14** as a white solid, mp 134-136 °C. ¹H NMR (CDCl₃, 300 MHz) d 8.4 (d, J = 2.2 Hz, 1H); 8.1 (d, J = 8.2 Hz, 1H); 7.7-7.6 (m, 1H); 4.8 (s, 2H); 2.7 (q, J = 7.4 Hz, 2H); 2.2-2.1 (m, 1H); 1.4 (s, 3H); 1.3 (t, J = 7.4 Hz, 3H); 1.0 (d, J = 6.9 Hz, 3H); 0.9 (d, J = 6.9 Hz, 3H).

The same procedures were used, starting with ethyl 2-(5-ethyl-2-pyridyl)-4-isopropyl-4-methyl-5-oxo-2-imidazoline-1-valerate instead of the acetate **13,** to prepare 2-(5-ethyl-2-pyridyl)-4-isopropyl-4-methyl-5-oxo-2-imidazoline-1-valeric acid **15**.

Figure 4 shows haptens used as competitors in indirect and direct EIAs. Hapten **16** (imazapyr-5-propenoic acid) was prepared as described by Wong and Ahmed (Wong, R. B. and Ahmed, Z. H. 1992. Development of an enzyme-linked immunosorbent assay for Imazaquin herbicide. *J. Agric. Food Chem.* 40: 811-818). Hapten **17** (imazapyr-5-formic acid) was synthesized by the procedure of Wong, et al. (Wong, R. B., et at. 1994. Functionalized imidazolinone haptens and protein conjugates thereof useful in assays for detection of imidazolinone herbicides. *U. S. Patent 5,342,771)* Structures **18**, **19**, **20**, and **21** are hapten-biotin conjugates. Their synthesis is described below in Procedure 2.

Compounds **7**, **8**, **11**, **15**, **16**, and **17** were purified by flash chromatography with 2;1 hexane/ethyl acetate eluent.

As noted above, three different classes of hapten that differed in spacer arm position and length were prepared for this study. Haptens **7** and **8** had spacers on the carboxylic acid group of the pyridine ring, while haptens **11** and **15** had spacers on the imidazole ring. All of these haptens were designed to mimic the ethyl-substituted pyridine moiety which is the distinguishing feature of imazethapyr. The substituents on haptens **16** and **17** replaced the ethyl moiety of the imazethapyr. These haptens were expected to bind antibodies that primarily recognized the imidazole ring characteristic of this entire class of herbicides, and were used for monoclonal antibody screening.

### Procedure 2: Conjugation of Haptens to Carrier Proteins and Biotin

A sample of hapten **7** 1.6 mg (4 µmol) was dissolved in 100 µl of tetrahydrofuran and diluted to 0.5 mL with an aqueous buffer (pH 7.2-7.6). 1-ethyl-3-(3-dimethylaminopropyl) carbodiimide hydrochloride (ECDI; 8.8 mg. 0.04 mmol) was added to activate the carboxyl groups on the hapten for two hours at room temperature. The mixture containing the activated hapten was slowly added to a solution of keyhole limpet in aqueous buffer (pH 7.2-7.6). The final molar ratio of hapten to total protein amino groups was greater than 100 to 1. The reaction mixture was stirred for 2 hr at room temperature arid then overnight at 4°C. Excess reagents were removed by gel filtration. The hapten **7**-KLH conjugate eluted in the void fraction was collected. It was passed through a 0.22µ filter and stored at 4°C. This conjugate was used for immunization. Hapten density was not determined except that the absorption spectra differences between the native protein and the protein conjugate were established.

Two other immunizing conjugates were made by conjugating haptens **7** and **15**, which were designed to present the ethyl pyridine moiety, the distinguishing feature of imazethapyr, most distal to the spacer arm, to cationized bovine serum albumin (cBSA; BSA treated with ethylenediamine to introduce aminoethyl amide groups). Conjugate formation could be demonstrated from UV spectra, but it was not possible to accurately quantify hapten densities. The cBSA conjugates showed a greater tendency to aggregate at 4 °C than KLH conjugates. Haptens **8**, and **11** were conjugated to unmodified BSA using the same ECDI activation method. Conjugates of haptens **7**, **8**, **11** and **15** to KLH and cBSA were used to immunize, as described below in Procedure 4.

Haptens **16** and **17** were conjugated to hydrazide derivatives of BSA and ovalbumin from Molecular Probes, Eugene OR, in neutral pH and purified by gel filtration as before. Haptens **16** and **17** were also reacted with biotin-LC hydrizide at room temperature in DMSO to give conjugates **18** and **19**, respectively. Imazethapyr and imazaquin analogs with carbon chain extensions from the nicotinic acid were also were also coupled to NHS-LC-biotin through their carboxylic acid groups to give conjugates **20** and **21**, respectively.

### Procedure 3. Enzyme Immunoassay Methods

EIAs were done essentially as described by **Karu *et al* (1994)**. For indirect EIAs, hapten-protein conjugates were adsorbed to wells of Immulon 2™ 96-well plates (Dynatech) in 0.1 mL of coating buffer (0.015 M Na₂CO₃ ― 0.035 M NaHCO₃ ―0.003 M NaN₃, pH 9.6). PBS-Tween containing 0.05% gelatin, 0.45 micron-filtered, was used as the diluent for antibodies and antibody-analyte mixtures, and as a blocking solution to prevent adventitious binding. The detecting antibody for the indirect EIAs was alkaline phosphatase conjugated goat anti-mouse IgG and IgM The substrate solution for color development was *p*-nitrophenyl phosphate 1 mg/mL, in 10% (w/v) diethanolamine-HCl, pH 9.8 ― 0.5 mM MgCl₂ ― 3 mM NaN₃.
*1. Indirect competition EIA*. Indirect competition EIA (immobilized hapten conjugate) was used to evaluate the responses of mice, screen the MAbs, and quantify imidazolinone analytes. Optimal amounts of imidazolinone-BSA or -OVA conjugate for plate coating and dilutions for the hybridoma culture supernate or antiserum were determined by checkerboard assay. To perform the assay, wells were coated overnight at 4°C with a limiting amount of conjugate in 0.1 mL of coating buffer. Imidazolinone standards (0.5 to 5000 ppb) or unknowns were mixed with a limiting amount of antiserum or hybridoma culture fluid in the diluent and incubated overnight at 4°C in tightly sealed polypropylene tubes. The coated wells were washed and blocked with 0.2 mL of 0.05% gelatin in PBS-Tween for 30 minutes at room temperature. After another wash, aliquots (0.1 mL) of the antibody-analyte mixture were added to wells, and the remainder of the assay was performed as previously described (Karu et al., 1994).
*2. Direct competition EIA*. Direct EIAs were performed by capturing the MAb on microwells and allowing the analyte to compete with a HRP-conjugated hapten for binding. Bound HRP-conjugate was then detected with a commercial HRP substrate. The sensitivity of this assay was enhanced by use of a commercial enzymatic signal amplification kit (GIBCO-BRL). Wells were coated with 200 or 500 ng of affinity-purified goat anti-mouse IgG or anti-mouse IgG Fc in the coating buffer at 4°C overnight. The next day, the wells were washed and blocked with PBS-Tween-gelatin for 30 minutes at room temperature, and then incubated for 1.5 hours at room temperature with hybridoma culture supernate. The plates were washed with PBS-Tween, and 0.1 mL of diluent containing the analyte and a limiting amount of biotinylated imidazolinone was added for 1.5 h at room temperature. The wells were washed three times with PBS-Tween, and Extravidin-alkaline phosphatase conjugate (Sigma) was then added for 30 minutes at room temperature. The plates were washed again with TBS (0.05 M Tris-HCl, pH 7.5,― 0.15 M NaCl) to remove remaining inorganic phosphate, which can inhibit the GIBCO-BRL EIA Amplification system. Substrate solution for the amplification step (0.05 mL) was added for 15 minutes at room temperature, followed by 0.05 mL of the enzymatic amplifying solution and chromogen. Color development at 492 nm was recorded.
*3. EIA Data Analysis*. Color development was monitored on a Multiskan EIA reader (Flow Laboratories) interfaced with a Macintosh computer, and the rates of the reaction were calculated by linear regression. Competition EIA dose- response curves were fitted with a 4-parameter logistic equation (Karu et al., 1994).

### Procedure 4: Preparation of hybridomas

*1. Immunization and Monitoring of Mice*. Pairs of female Swiss Webster and Biozzi mice were immunized with KLH and cBSA conjugates of hapten **7** and hapten **15**. The initial dose was of 50 µg of conjugate (as carrier protein) in 0.1 mL of physiological saline, and one mouse dose (approx. 50 µg) of Ribi adjuvant ("MPL + TDM Emulsion," Ribi Immunochem Research, Inc., Hamilton, MT). Boosts of 25 µg of conjugate protein in saline with Ribi adjuvant were given 7 and 22 days after the first dose. All immunizations were subcutaneous, in 3 or 4 sites on the back of the mouse. Sera were taken on the 29th day after the first injection, titers were determined by indirect EIA, and the sera were then analyzed for ability to bind free imudazolinones in an indirect competition EIA. The best-responding Swiss Webster mouse was given a final boost by tail vein injection of 50 µg of hapten 7-KLH in saline, 4 days prior to cell fusion and about 60 days after the first immunization. To reduce the likelihood of anaphylactic or delayed-type hypersensitivity responses, a subcutaneous injection of antihistamine and anti-vasospasm drugs was administered 1 h before the intravenous hyperimmunizing boost (Karu, A. E. 1993. Monoclonal antibodies and their use in detection of hazardous substances. In: *Hazard Assessment of Chemicals* ― *Current Developments*. *vol. 8*, eds. J Saxena, pp. 205-321. Washington, D.C.: Taylor & Francis Intl. Publishers).
   All sera that bound to one or more coating antigens were tested for competitive binding of imazethapyr in solution. Sera from Swiss Webster mice immunized with hapten **7** conjugates competitively bound free imazethapyr in these EIAs. Spleen from the mice immunized with hapten **7**-KLH with the lowest I₅₀ values was used to derive the hybridomas.
*2. Hybridoma production, selecting and archiving*. The "complete medium" for hybridoma culture was Iscove's Modified Dulbecco's Medium (IMDM), supplemented with 20% (v/v) fetal bovine serum (FBS), 10 µg/mL kanamycin sulfate, 5 x 10⁻⁵ M β-mercaptoethanol, and 1 µg/mL of 1/3 iron-saturated transferrin (human Type III, Sigma Chemical Co.), and Iscove's lipid emulsion (Iscove, N. 1984. Culture of lymphocytes and hemopoietic cells in serum-free medium. In: Methods for Serum-Free Culture of Neuronal and Lymphoid Cells. eds. D Barnes, D Sirbasku, G Sato, pp. 169-185. New York: Alan R. Liss; Iscove, N. and Melchers, F. 1978. Complete replacement of serum by albumin, transferrin, and soybean lipid in cultures of lipopolysaccharide-reactive B lymphocytes. J. Exp. Med. 147: 923-933).
   Hybridomas were selected in complete medium containing 10% (v/v) J774A.1 macrophage-conditioned medium (Sugasawara, R. J., et al. 1985. The influence of murine macrophage-conditioned medium on cloning efficiency, antibody synthesis, and growth rate of hybridomas. J. Immunol. Methods 79: 263-275), 10⁻⁴ M hypoxanthine, 8 x 10⁻⁷ M aminopterin, and 3 x 10⁻⁵ M thymidine ("HAT"), expanded, and frozen as described by Karu, A. E., et al. 1994. Synthesis of haptens and derivation of monoclonal antibodies for immunoassay of the phenylurea herbicide diuron. J. Agric. Food Chem. 42: 301-309.
   Hybridomas were prepared by electrical fusion with P3X63 AG8.653 myelomas as the fusion partner (Forghani, B. and Karu, A. E. 1993. Monoclonal Antibodies. In: *Virology LABFAX*, D R Harper, ed., pp. 187-214. Oxford, UK: Bios Scientific Publishers Ltd.; Karu et al, 1994). The hapten-specific cultures were expanded and 11 cultures were competitively bound the imidazolinones in competition EIAs. Ten of these were subcloned by limiting dilution. Three MAbs that bound hapten but did not competitively bind free imazethapyr were also saved. Selected clones of MAbs identified as 1A5, 2C6, 3A2, 3A5, and 4A6 were subsequently expanded as described previously, to produce culture medium and ascites fluid sufficient for several thousand assays (Karu et al., 1994). Immunoglobulin subclass was determined using a commercial EIA (Southern Biotechnology Associates). IgG antibodies were purified by affinity chromatography on Protein A or Protein G columns. Fab fragments were prepared by digestion of affinity-purified IgG with immobilized papain and removal of the Fc fragments on Protein A-sepharose using an ImmunoPure Fab Preparation Kit (Pierce Chemical Co., Rockford IL) according to the manufacturer's instructions.

### Results: Properties of the MAbs

Different specificities were evident not only between MAbs, but for individual MAbs using two different coating conjugates. Five most interesting and sensitive clones were selected and archived. Selected clones of these five cell lines were expanded and archived. The immunoglobulin subclasses of these MAbs and their selectivity for imidazolinone herbicides are shown in Table 2, Figure 5. MAbs 1A5, 1D2, and 2C6 were most sensitive for imazethapyr. MAb 3A2 was most sensitive for imazaquin, and MAb 3A5 was most sensitive for imazamethabenzmethyl. Imazethapyr and imazapic could be distinguished by responses of MAbs 1D2 and 3A2. Imazamox was sensitively detected by MAbs 2C6 and 3A2, and could be distinguished from the other compounds by use of three or more MAbs and pattern recognition analyses as described below (see Applications, below).
*1. Binding Kinetics*. The equilibrium dissociation constants (K_{D}) for MAbs 3A2 and 3A5 were measured by kinetic exclusion fluoroimmunoassay (KinExA) (Blake, D. A., et al. 1997. Characterization of a metal-specific monoclonal antibody. In*: Immunochemical Technology for Environmental Applications (ACS Symposium Series 657)*, eds. DS Aga, EM Thurmann, pp. 49-60. Washington, D.C.: American Chemical Society.), and also in a Pharmacia BIAcore surface plasmon resonance instrument (Malmborg, A.-C. and Borrebaeck, C. A. K. 1995. BIAcore as a tool in antibody engineering. *J. Immunol. Methods* 183: 7-13.). Since the hapten conjugates used in these rate studies were racemic mixtures, the dissociation constants obtained can only be a median rate for the different chiral isomers. Both methods placed the K_{D} of MAbs 3A2 and 3A5 for imazethapyr between 10 and 100 nM, as summarized in the table below:

| | BIAcore K_{D} (nM) | | KinExA K_{D} (nM) |
|---|---|---|---|
| Hapten conjugate --> | **16**-OA | **17**-OA | **16**-BSA |
| MAb | | | |
| 3A2 | 24.7 | 51.6 | 11.4 |
| 3A5 | 9.6 | 10.8 | 73 |

The equilibrium constants measured by KinExA and BIAcore confirmed indications from the competition EIAs that MAbs 3A2 and 3A5 are of moderately high affinity. The KinExA results were considered to be more valid, primarily because they were direct measurements of unoccupied antibody from solutions at analyte-antibody equilibrium. Differences between the values determined with the two instruments were due in part to the differences in procedures. The KinExA directly measures free antibody in solution equilibrium by trapping it on beads coated with hapten conjugate. The BIAcore computes the K_{D} from the antibody's association and dissociation with hapten conjugate immobilized on the sensor chip. Others have reported that BIAcore gives low estimates of the rate constants because of slowed diffusion and re-binding in the hydrogel that forms the solid phase on the sensor chip (Nieba, L., et al. 1996. Competition BIAcore for measuring true affinities: large differences from values determined from binding kinetics. *Anal. Biochem.* 234: 155-165.; Schuck, P. 1996. Mass transport and kinetic analysis of ligand-receptor interactions as detected with an evanescent wave biosensor. *Biophys. J.* 70: A212 (1230-1249). However, because the errors in on- and off-rate estimates are similar, their effects cancel in the K_{D} values computed from them.
*2. MAb Protein sequencing*. MAb 3A5 IgG was affinity purified on Protein A Sepharose by a standard method (Harlow, E. and Lane, D. 1988. *Antibodies: A Laboratory Manual*, Cold Spring Harbor, NY: Cold Spring Harbor Laboratory. pp. 726). Purified protein was subjected to SDS electrophoresis. The proteins were electrophoretically transferred from the gel to polyvinylidene fluoride membrane and stained with Coomassie Blue in methanol:acetic acid::1:1 to visualize the IgG H and L chain bands. Small rectangles containing the stained bands were cut from the membrane. Automated sequencing was done on these samples (University of Kentucky Macromolecular Structure Analysis Facility, Lexington, KY) to determine the first 15 N-terminal amino acids. The N-terminal 15 amino acids of the MAb 3A5 H and L chains were determined to be as follows in Table 4: 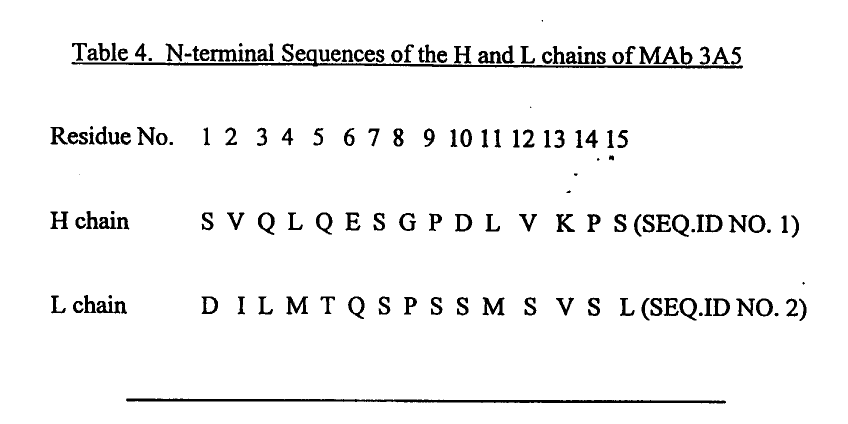
*3. Selectivity for Imidazolinones* The specificity of MAb 3A2 for the imidazolinone class of compounds was demonstrated by testing with sixteen commonly-available-agrochmeicals in an indirect competition EIA (wells coated with hapten **17**-OA). No cross-reactivity was observed with up to 10 ppm of acifluorfen, alachlor, atrazine, primisulfuron, EPTC, glyphosate, metolachlor, molinate, oxyfluorfen, paraquat, pendimethalin, phenmedipham, sethoxydim, or thiobencarb. MAb 3A2 also did not cross-react wit up to 5 ppm of chlorimuron ethyl or chlorsulfuron, but 5-10 ppm of these herbicides gave 10-15% inhibition. Thus the EIA for imidazolinone residues was not affected by any of the major herbicides that may be found in environmental samples.
*4. Stability in Organic Solvents*. Since many standard imidazolinone residue recovery and assay and separation procedures require methanol or acetonitrile, the effect of these solvents on the indirect EIA was tested. MAbs incubated overnight at 4° in PBST wit various amounts of solvent were tested for ability to bind hapten conjugate in the indirect EIA. MAb 3A2 binding was actually improved in 5-25% methanol. Binding of MAbs 1D2, 2C6, and 3A5 to the **16**-BSA and **17**-OA conjugates progressively decreased in increasing methanol, retaining about 50% binding activity after exposure to 20% methanol. MAb 1A5 binding to the **11**-OA conjugate was much more sensitive. The results of the methanol stability tests are shown in Figure 6A. All of the MAbs were more sensitive to acetonitrile than methanol, as illustrated in Figure 6B. The relative sensitivities of each MAb to acetonitrile and methanol were similar. Although the overnight exposure to the solvents was harsher than necessary for indirect EIAs, it demonstrated that MAbs 3A2 and 3A5, and to a lesser extent 1D2 and 2C6, potentially can be used for assays in as much as 15% methanol or 5% acetonitrile.
*5. Stability of Conjugate-coated microplates for imidazolinone EIA*. For intermittent use of immunoassays and for assay kit manufacture, it is advantageous to be able to coat substrates (i.e., plates or beads) and store them until they are needed. Immulon 2™ plates were coated for indirect EIA with optimal amounts of 11-OA conjugate (316 ng/well for MAb 1A5), or 17-OA (10 and 32 ng/well for MAbs 3A2 and 3A5, respectively), and stored containing the coating buffer, blocking buffer, or dry at -80 °C. Plates were removed after 8, 16, and 30 days and compared with freshly coated plates by performing indirect EIAs. The plates stored in coating buffer gave the best results, with consistent response rates at the low-dose asymptote, I₅₀ values, and agreement between replicate samples (data not shown). The least consistent results were obtained with coated wells stored containing the blocking buffer. More sensitive assays, i.e., lower I₅₀ values, were obtained with plates stored for 16 and 30 days than with plates that were freshly coated or stored for 8 days. These results indicated that it is feasible to store pre-coated substrates for periods of at least 30 days, with no loss, and even some improvement of performance.
*6. Chiral specificity of MAbs*. The R isomer of imazethapyr is known to be about ten times more inhibitory to acetohydroxyacid synthase than the S isomer (Stidham, M. and Singh, B. 1991. Imidazolinone-acetohydroxy acid synthase interactions. In: The Imidazolinone Herbicides, eds. DL Shaner, SL O'Connor, pp. 71-90. Boca Raton, FL: CRC Press). The immunizing hapten was a racemic mixture of the R and S isomers at the 4' carbon on the imidazole ring. Indirect competition EIAs were done with pure R and S isomers and a technical mixture as competitor. The results, summarized in Table 3 (Figure 7), show that MAbs 3A5, 1D2, and 1A5 preferentially recognized the S isomers of the major imidazolinones. MAbs 3A2 and 2C6 bound either isomer about equally well, within the range of experimental variation.

### II. Derivation and Properties of a Single-Chain Fv Antibody to Imidazolinone Herbicides

*Introduction.* Recombinant antibodies consist of the sequences of the heavy and light chain variable domains (abbreviated V_{H} and V_{L}) that bind the target analyte (in this instance, an imidazolinone herbicide). These sequences may be reassembled on either side of a short linker sequence to produce DNA encoding a single polypeptide, V_{H}-linker-V_{L}, that folds to form a functional ligand-binding scFv antibody (Hoogenboom, H. R, et al. 1992. Building antibodies from their genes. Immunol. Rev. 130: 41-68; Winter, G. and Milstein, C. 1991. Man-made antibodies. Nature 349: 293-299). In a similar way, the V_{H} and V_{L} domains along with their adjacent constant domains (V_{H}―C_{Hl} and V_{L}―C_{L}) can be cloned and expressed so that they form a disulfide-linked functional antibody known as a recombinant Fab, because it resembles the Fab fragments tat can be prepared from whole immunoglobulin by digestion with the protease papain. Simplified diagrams of these antibody forms are shown in Figure 8 for comparison.

To obtain the imidazolinone-specific scFv 3A5 in accordance with this invention, the V_{H} and V_{L} sequences were derived from the messenger RNA of the 3A5 hybridoma cells, and cloned into a phagemid vector, primarily using established techniques and a commercial kit as described below. A schematic of major steps in the derivation of the scFvs in accordance with the preferred embodiment of the present invention described in this example is provided in Figure 9.

*Bacterial media*. SOBAG medium contained 20 g of Bacto-tryptone, 5 g Bacto yeast extract, and 0.5 g NaCl per liter, 0.01 M MgCl₂, 0.11 M glucose, and 100 µg/mL ampicillin. Super Broth was previously described (Scholthof et al., 1997). M9 minimal medium consisted of M9 salts with 2 mg/mL thiamine, 1 mM MgSO₄, and 0.2% casainino acids. All other media, agar plates, antibiotics, and supplements were prepared as described in Maniatis, T., et al. 1989. *Molecular Cloning, A Laboratory Manual,* 2nd ed. Cold Spring Harbor, NY: Cold Spring Harbor Laboratory, and the instructions in Pharmacia's Recombinant Phage Antibody System (Pharmacia Biotech Corp. 1994. *Mouse ScFv Module/ Recombinant Phage Antibody System Instruction Manual,* Piscataway, NJ: Pharmacia Biotech).

*Haptens and Conjugates*. Imidazolinone reference standards, purified *R* and *S* stereoisomers of imazethapyr and imazaquin, and hapten conjugates were provided by Cyanamid Agricultural Research Center, Princeton NJ. The haptens used in this example are shown in Figure 10. Syntheses of haptens **7, 11, 16,** and **17** were described above, with reference to Figures 1 through 4. Imazethapyr was reacted with 3-(2-aminoethanamido)propanoic acid to produce a hapten identical to that in the LC-biotin conjugate **20** shown in Figure 4. For consistency, this is referred to as hapten **20** 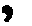 in Figure 10. Haptens **20** 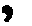 **, 22, 23,** and **24** were synthesized and characterized at the Cyanamid Agricultural Research Center. Conjugates of these haptens with ovalbumin, calf intestine alkaline phosphatase (AP), and horseradish peroxidase (HRP) were prepared using published methods.

*Analytical Methods*. Amounts of mRNA and DNA were determined by UV spectrophotometry. DNA was sequenced using a Commercial kit (Sequenase 2.0 - US Biochemicals, Cleveland, OH) and a-[³⁵S]dATP (Amersham, Arlington Heights, IL). Sequences at the ends of the V_{H} and V_{L} regions were determined using the synthetic oligonucleotide primers recommended by Pharmacia. Additional primers were designed from the sequences and synthesized by the U.C. Berkeley Molecular and Cell Biology Division's DNA synthesis facility. Protein concentration in crude bacterial lysates was estimated using the formula *c*_{*protein*} = 1.45*A*₂₈₀ - 0.74*A*₂₆₀ to correct for the presence of nucleic acid (Fischer, L. 1969. An introduction to gel chromatography. In: Laboratory Techniques in Biochemistry and Molecular Biology, edited by TS Work, E Work, pp. 365-366 (Appendix 2). Amsterdam: North-Holland Publishing Co.). Concentrations of purified scFv were determined by BCA protein assay (Pierce Chemical Co., Rockford IL), or estimated from absorbance at 280 nm, using an extinction coefficient based on the amino acid composition (Manchester, K. L. 1996. Use of UV methods for measurement of protein concentrations. *BioTechniques* 20: 968-970). Denaturing polyacrylamide gel electrophoresis (SDS-PAGE) and western blotting methods were described previously (Scholthof, K.-B. G., et al. 1997. Derivation and properties of recombinant Fab antibodies to the phenylurea herbicide diuron. *J. Agric. Food Chem.* 45: 1509-1517). Bands were detected on western blots by incubation with anti-E tag or FLAG tag MAb followed by goat anti-mouse IgG-alkaline phosphatase conjugate. The scFv constructs with the strep-tag were detected with streptavidin-alkaline phosphatase conjugate.

*Preparation of conjugate-coated magnetic beads for phage panning*. 0.2 mg/mL of imidazolinone hapten **16**-BSA conjugate was covalently coupled to carboxylated magnetic beads by a modification of the two-step procedure provided by the manufacturer, as previously described (Scholthof et al., 1997). For phage panning, 20 µL of this suspension gave visible pellets when the tubes were placed in the magnetic attractor. The conjugate-coated beads were tested by EIA to verify specificity for the anti-imidazolinone MAb. The beads were incubated with MAb 3A2.2 ascites fluid or non-immune mouse serum (NMS) in TPG buffer (0.05 M NaH₂PO₄, 0.1% NaCl, 2 mg/mL gelatin, pH 6.6), washed, and then incubated with alkaline phosphatase-labeled goat anti-mouse Ig in TPG, and washed again with TPG. Alkaline phosphatase substrate was added and color development was monitored. Beads coated with unmodified BSA or NMS gave no significant binding of MAb 3A2 (ΔA_{405 nm} = 0.07 and 0.02 respectively), but the haptenated beads bound the MAb ( ΔA_{405 nm} = 0.58).

*Preparation of immunoglobulin mRNA and cDNA*. Messenger RNA was isolated from 5 x 10⁶ log-phase cells of hybridoma cell line 3A5.1. The polyadenylated mRNA was purified using an oligo(dT)-cellulose spin column provided in the QuickPrep kit according to the kit instructions (Pharmacia Biotech Corp. 1993. *QuickPrep mRNA Purification Kit Instruction Manual,* Piscataway, NJ: Pharmacia Biotech), and stored as an ethanol precipitate at -80°C. Single-strand complementary DNA (cDNA) was made from the mRNA using FPLCpure murine retroviral reverse transcriptase, random hexadeoxyribonucleotide primers, and other reagents provided in the Pharmacia scFv kit. Half of the cDNA was used in the subsequent polymerase chain reaction (PCR) step to produce a V_{L} domain repertoire, and half was used to make a V_{H} domain repertoire:

*Production of phage displaying imidazolinone-binding antibodies*. The scFv gene fragment was produced by PCR in four steps, following the Pharmacia protocols (Pharmacia, 1994) with some modifications. The first two PCR reactions amplified the V_{H} and V_{L} segments, respectively. A third PCR step assembled the V_{H} chain product, V_{L} chain product and linker-primer DNA fragments to form the scFv construct. This full-length scFv fragment was amplified, and *Sfi* I and *Not* I restriction sites were added in a final PCR. This was purified by agarose gel electrophoresis and ligated into the pCANTAB 5E vector DNA which had been cut with *Sfi* I and *Not* I, and electrophoretically purified (Maniatis et al., 1989). The resulting construct was used to transform competent cells of *E. coli* strain TG1. The transformation method of Hanahan, D. 1985. Techniques for transformation of *E. coli.* In: *DNA Cloning: A Practical Approach,* eds. DM Glover, pp. 109-121. Oxford, UK: IRL Press, was used instead of the protocol provided by Pharmacia, after initial experiments indicated that the Pharmacia procedure was less efficient. Transformed TG1 cells were grown at 37°C as a broth culture rather than as colonies on agar. Replication of display phage was induced by adding M13K07 helper phage to a log-phase culture (10-200 mL) of transformed cells. The culture was propagated overnight with shaking at 37 °C, and phage were harvested by polyethylene glycol-NaCl precipitation (Barbas, C. F. and Lerner, B.. A. 1991. Combinatorial immunoglobulin libraries on the surface of phage (phabs): Rapid selection of antigen-specific Fabs. *METHODS: A Companion to Meth. Enzymol*. 2: 119-124; Pharmacia, 1993a).

*Phage selection and enrichment*. All steps were done at room temperature. Phage displaying imidazolinone hapten-binding scFv were selected essentially as described by Scholthof *et al* (1997). Phage concentrated from a 10 mL culture were resuspended in 0.3 mL of TPG buffer. The suspension was clarified by centrifugation and aliquots (0.1 mL, 1-3 x 10¹¹ cfu/mL) were incubated with 0.08 mL of TPG and 0.02 mL of **16**-BSA conjugate-coated magnetic bead suspension for 30 min on a roller. Beads were sequestered with a magnet, and the phage suspension was discarded, and the beads were washed five times for 3 min with 1 mL of TPG. Imazethapyr [0.1 mL of 1 part per million (1 µg/mL) in TPG] was added, bound phage were eluted for 10 min on the roller, and the beads were sequestered with the magnet. Aliquots of the eluted phage were used to infect 10 mL mid-log phase *E. coli* TG1 broth culture. Helper phage was added and the phage were amplified overnight with shaking at 37°C according to standard procedures (Barbas and Lerner, 1991). This amplification and panning cycle was repeated two more times.

*E. coli* TG1 cells were infected with phage recovered after the third panning, and grown on selective agar. Individual colonies were aseptically picked and transferred into wells of a sterile cell culture plate that contained 0.1 mL of culture medium. This plate became the "master plate." The master plate was shaken overnight at 30°C, and 0.02 mL was then transferred from each well into wells of a sterile 96-well polyvinyl chloride "V" bottom plate containing helper phage (approx. 1.05 x 10¹⁰pfu/mL) in 0.2 mL of culture medium. The master plate was sealed and stored at 4°C. The replica "V" bottom plate was incubated at 37°C for 2 hr, then centrifuged on a microplate carrier (2,000 x g, 10 min, in an IEC PR-6000 centrifuge). The supemates were removed aseptically, and the helper phage-infected cell pellets were resuspended by adding 0.2 mL of growth medium to each well. This plate was incubated at 37°C overnight with shaking, the cells sedimented by centrifligation, and the supemates (clones of display phage) were then harvested and tested for ability to bind to imidazolinone hapten in a phage EIA.

*Phage EIA*. An indirect EIA was performed essentially as described previously (Karu, et al. 1994). Immulon 2™ microwells were coated overnight at 4°C with 16-BSA (300 ng/well). All subsequent steps were at room temperature. The coating solution was discarded, the plates were washed three times with PBST, and wells were blocked for 30 min wit 0.45 µm-filtered 2% nonfat dry milk in PBST (PBST-NFDM). This blocking buffer was removed, and each well received 0.01 mL of phage suspension from the corresponding well of the V-bottom plate described above, in 0.1 mL of the blocking buffer. After 2 h, the phage suspensions were aspirated off, the wells were washed three times wit PBST, and rabbit anti-M13 phage serum (1:500 in 0.1 mL blocking buffer) was added for 1 h. Negative controls included supernates from helper phage-infected TG1 harboring the pCANTAB 5E vector with no insert, TG1 with the pUC18 phagemid, and phage displaying the diuron-specific recombinant Fab 224 (Scholthof et al, 1997). The plates were again washed three times with PBST, incubated for 1 h more with alkaline phosphatase-conjugated goat anti-rabbit IgG (1:500 in 0.1 mL blocking buffer), washed, and developed with alkaline phosphatase substrate. Rates of color development at 405 nm were read on a Flow Multiskan EIA reader.

*Production of soluble scFv*. Log-phase HB2151 cells were infected with phage EIA-positive clones from the master plate. These cultures were streaked on SOBAG agar plates containing 100 µg/mL of nalidixic acid, which selected the nalidixic acid-resistant HB2151 transductants and eliminated the possibility of introducing infected TG1 cells (Pharmacia, 1993). Colonies were grown to mid-log phase (A_{550 nm} = 0.3-0.5) in Super Broth containing 0.4% glucose and 100 µM carbenicillin. ScFv production was then induced by addition of isopropyl β-D-thiogalactopyranoside (IPTG) to a final concentration of 50 µM. The induced cultures were grown at 30°C overnight. A portion of the culture medium was saved for analysis, and the cells were collected by centrifugation (15 min, 5,000 x g) for preparation of extracts.

Whole-cell extracts for SDS-PAGE were prepared by resuspending a portion of cells in SDS-PAGE sample buffer in a microfuge tube, heating to 100°C for 5 min, and removing the cell debris by centrifugation. Concentrates of culture medium were prepared by precipitation with 80% ammonium sulfate, resuspension in 0.01 of the original volume, and dialysis. Periplasmic lysates were prepared by three different procedures: (a) the method recommended by Pharmacia Corp., in which the cells are lysed in PBS-1 mM EDTA (Pharmacia, 1993); (b) sequential lysis with lysozyme, EDTA, and Brij 58 surfactant (Karu, A. E. and Belk, E. D. 1982. Induction of E. coli recA protein via recBC and alternate pathways: quantitation by enzyme-linked immunosorbent assay (ELISA). *Molec. & Gen. Genetics* 185: 275-282); and (c) cell suspension in a spheroplast buffer (100 mM Tris-HCl, 0.5 M sucrose, 0.5 mM EDTA, pH 8.0) and lysis with lysozyme (Minsky, A., et al. 1986. Secretion of beta-lactamase into the periplasm of Escherichia coli: evidence for a distinct release step associated with a conformational change. *Proc. Natl. Acad. Sci. USA* 83: 4180-4184). One tablet of Complete Mini combination protease inhibitor was added to each 10 mL of the buffers used to make these extracts. The scFv was concentrated from lysates and culture media by precipitation between 40% and 80% ammonium sulfate (v/v). The concentrated protein was dialyzed against 3 changes of 100 volumes of TBS (50 mM Tris-HCl, pH 7.4- 150 mM NaCl) containing protease inhibitors, and 0.2 µm-filtered. These preparations were either affinity purified as described below, or they were stored at -20 °C with 20% (v/v) glycerol added.

*Enzyme Immunoassay Methods*. Indirect and direct EIA methods were carried out as previously described (Chin et al., 1998; Karu et al., 1994), with modifications. To detect functional soluble scFv, plates were coated with 300 ng/well 16-BSA. The wells were incubated first with *E. coli* periplasmic lysates (1:10 in PBST-NFDM), then with mouse MAb specific for the E-tag epitope (Pharmacia Biotech; 1 µg/mL in PBST-NFDM), and finally with alkaline phosphatase-conjugated goat anti-mouse IgG, with washes between each incubation. Indirect competition EIAs were done on plates coated with 100 ng of **16**-BSA. Imidazolinone reference standards in 0.1 mL of PBST-NFDM were mixed with an equal volume of periplasmic lysate (1:10 in PBST-NFDM) containing scFv, incubated overnight at 4 °C, then 0.1 mL was added to each well for 2 h at room temperature. The plates were processed further as described above. For direct EIA to capture scFv from periplasmic extract, plates were coated with 500 ng/well of affinity-purified goat anti-mouse IgG, blocked with PBST-NFDM, incubated with 200 ng/well of anti-E tag MAb, and then washed. Periplasmic lysates (1:10 or 1:100 in PBST-NFDM were applied for 2 h, the plates were washed, and then Incubated with hapten-enzyme conjugate (**11**-AP or **7**-AP; 1:1,000 in PBST-NFDM). Color development was monitored on a Multiskan EIA reader (Flow Laboratories) interfaced with a Macintosh® computer, and the rates of the reaction (?A_{405 nm} /min x 10³) were calculated by linear regression. Competition dose-response curves were fitted with the 4-parameter logistic equation using Passage II software on a Macintosh computer.

*DNA sequencing*. p3A5.1 c7 phagemid DNA was prepared from cultures of *E. coli* XL-1 Blue after it was found that this strain gave higher quality DNA for sequencing than HB2151. Both DNA strands were sequenced by the dideoxynucleotide chain termination method. The sequences were obtained using primers recommended by Pharmacia, and others synthesized by the U.C. Berkeley Molecular and Cell Biology Division's DNA synthesis facility. Several short AT-rich and GC-rich segments of the DNA that were not resolved initially were sequenced with the procedure described by DeShazer, D., et al. 1994. Boiling eliminates artifact banding when sequencing double-stranded templates. *BioTechniques* 17: 288-289, using terminal deoxynucleotidyl transferase. The nucleotide sequences and open reading frames were collated and analyzed using MacVector and AssemblyLIGN software (Eastman Kodak, Rochester NY).

### Results and Discussion

*Selection of phage displaying imazethapyr-specfic scFv*. Phage panning was monitored by determining infective titer of the phage suspensions before and after each round. The phage titer before the first, second, and third pannings was 2.8 x 10¹¹, 3.5 x 10¹¹, and 1.0 x 10¹¹ cfu/mL, respectively. The phage titers recovered after these pannings were 1.3 x 10⁷, 1 x 10⁹, and 2 x 10⁹ cfu/mL, respectively. The titer of phage that bound to unconjugated BSA after the first panning was about 1 X 10⁷ cfu/mL. These results indicated progressive enrichment of the phage populations, and suggested that at least 2% of the phage eluted from the third panning should display scFv specific for the **16**-BSA hapten. Seven of 24 phage clones from the third panning gave responses several times greater than negative controls in the EIA. Soluble scFv was produced from each of the four phage clones that gave EIA rates greater than 2.5.

*Expression of soluble scFv*. The scFv, with a mass of 26.67 kDa computed from its amino acid composition, consistently migrated with an apparent molecular weight of 30.6 kDa in SDS-PAGE. On western blots, extracts of boiled whole cells, periplasmic lysates, and culture medium from IPTG-induced cells of all four clones produced a single immunostaining band at 30.6 kDa. Periplasmic lysates contained the largest amount of active scFv with the fewest contaminants. The periplasmic lysis method described by Minsky, et al. gave the greatest yield and purity of p3A5.1c7. Virtually all of the active scFv was recovered from these fractions by precipitation between 40% and 80% ammonium sulfate. In an indirect EIA, scFv from periplasmic extracts of all four clones bound to **16**-BSA conjugate, but only one, p3A5.1 c7, competitively bound free imidazolinones. Figure 11 is the genetic map of this clone.

*Optimal Conditions for Induction and Lysate Preparation*. The amount and quality of recombinant antibody expressed in *E. coli* depends on several factors that include potential toxicity of the antibody to the bacteria, the rate of antibody synthesis relative to that of essential host proteins, and proper folding of the antibody (Byrne, F. R., et al. 1996. Cloning, expression, and characterization of a single-chain antibody specific for the herbicide atrazine. *Food & Agric. Immunol.* 8: 19-29; Knappik, A., et al. 1993. The effect of folding catalysts on the *in vivo* folding process of different antibody fragments expressed in *Escherichia coli. Bio/Technol.* 11: 77-83; Plückthun, A. 1991. Antibody engineering: Advances from the use of *Escherichia coli* expression systems. *Bio/Technol*. 9: 545-551; Plückthun, A. 1991. Strategies for the expression of antibody fragments in *Escherichia coli*. *METHODS: A Companion to Meth. Enzymol.* 2: 88-96; Tsumoto, K., et al. 1994. Effect of the order of antibody variable regions on the expression of the single chain single-chain HyHEL10 Fv fragment in *E. coli* and the thermodynamic analysis of its antigen-binding properties. *Biochem. Biophys. Res. Comm.* 201: 546-551). To maximize expression of functional p3A5.1c7 scFv, different media and inducer concentrations were tested in cultures grown at 30 °C and 37°C.

The relative yield of p3A5.1c7 scFv was measured in three different growth protocols. In the first procedure, recommended by Pharmacia, the cultures were grown at 30°C in rich medium (Pharmacia's SOBAG formula) containing 2% glucose prior to induction. The cells were then recovered by centrifugation and resuspended in glucose-free medium containing 1 mM IPTG. In the second procedure, the culture was grown in medium containing 0.4% glucose and induced by addition of 1 mM IPTG. The glucose was depleted by the time of induction, eliminating the need to recover the cells and transfer them into glucose-free medium. In the third method, the cultures were grown in M9 minimal medium, induced with much less IPTG (0.02-0.03 mM), and sucrose was added to 0.4 M in the medium after induction to promote correct folding and reduce aggregation of the scFv, as described by Sawyer, J. R., et al. 1994. The effects of induction conditions on production of a soluble anti-tumor sFv in *Escherichia coli. Protein Eng.* 7: 1401-1406. There were no significant differences in the amount of functional p3A5.1c7 obtained from cultures grown by these three protocols. It was also tested whether production was enhanced when media were supplemented with tap water to provide trace minerals. Formulation of the media with tap water instead of distilled water made no significant change in the amount of functional p3A5.1c7 produced.

Figures 12A and 12B compare the yield of functional p3A5.1c7 scFv from cultures at 30 °C in SOBAG medium as a function of IPTG concentration. Optimal induction required 0.05 -0.1 mM IPTG, and the yield was much lower when the cells were induced with 1 mM IPTG, according to the instructions in the scFv expression kit (Pharmacia, 1994). The lower IPTG concentration was also optimal for cultures in M9 minimal medium. More functional scFv was produced after growth and induction at 30°C than at 37°C. Growth overnight (8-12 h) at 30°C after induction produced approximately ten-fold more functional scFv in the periplasm than growth for only 3 h after induction, which was recommended in the Pharmacia kit instructions.

*Properties of p3A5.1 scFv.* The scFv competitively bound imidazolinones in indirect EIAs with **16**-BSA as coating conjugate, but it bound to **17**-OA conjugate too weakly for practical competition assays. By contrast, MAb 3A5.1, from which the scFv was derived, bound to **16**-BSA but gave the most sensitive competitive binding of imidazolinones in indirect EIAs with **17**-OA coating antigen. The I₅₀ values for imazethapyr and imazaquin with this scFv were about two-fold higher (less sensitive) than obtained with the MAb.

Like MAb 3A5, scFv p3A5.1 c7 was specific for the *S* isomers of imazethapyr and imazaquin in the indirect EIA, as summarized in Table 5 (Figure 13). Direct competition EIAs with two different hapten-HRP conjugates showed the same preference for the *S* isomers of imazethapyr and imazaquin, as shown in Table 6 (Figure 14). In both EIA formats, technical-grade mixtures of the herbicides competed with I₅₀ values between those of the pure *R* and *S* isomers. Because the technical mixtures were not racemic, relation of the I₅₀ to the mole fraction of *S* isomer was not attempted.

The relative affinities of the MAbs and affinity-purified scFv were determined by indirect EIA using equimolar amounts of protein, essentially as described by Graham, B. M., et al. 1995. Cloning, expression, and characterization of a single-chain antibody fragment to the herbicide paraquat. *J. Chem. Tech. Biotechnol.* 63: 279-289. The results are illustrated in graphical form in Figures 15A (**16**-BSA) and 15B (**17**-OA), with the MAb represented by the solid line and the scFv represented by the broken line in each plot. The results indicate that the affinity of the scFv for hapten **16** was about 1,000-fold poorer than that of the MAb, and the difference in affinities for hapten **17** was even greater.

*ScFv tolerance of organic solvent in the EIA.* The stability of the scFv and the MAb to organic solvents was tested using the indirect EIA. However, the large difference in affinities made it impractical to compare the scFv and MAb on a single hapten under conditions that could be used for competition EIAs. The scFv was significantly more stable than MAb 3A5.1 during incubation in PBS with methanol (up to 30% v/v) or acetonitrile (up to 10% v/v) for 1 hr at room temperature or overnight at 4 °C (Figure 16: Binding of scFv to 360 ng/well of **16**-BSA (--○--) and MAb 3A5 to 316 ng/well of **17**-OA (―●―) after incubation for 1 h at room temperature in PBS with the indicated amounts of methanol (A) or acetonitrile (B). Panel **C** shows relative activity of the scFv after incubation 16 h at 4 °C in PBS with the indicated amounts of methanol ―▲―) or acetonitrile (--●--). Ordinate is the ratio of EIA responses of sample at the indicated concentration of solvent and a sample in PBS with no solvent, X 100%. Triplicate samples are shown.). Incubation in PBS with 20% methanol or 5% acetonitrile actually improved binding of the scFv, as shown. Indirect competition EIAs could be done in PBS with up to 5% acetonitrile or 0.5% acetone, but sensitivity was reduced (I₅₀ values were increased).

*Sequence of the scFv.* The nucleotide and corresponding amino acid sequences of p3A5.1c7 are shown in Figure 17. The V_{H} coding region (339 bp) belongs to mouse immunoglobulin subgroup IA, and the V_{L} sequence (321 bp) is a member of the Kappa V subgroup (Kabat, E. A., et al. 1991*. Sequences of Proteins of Immunological Interest*, 5th ed. Washington, D. C.: Public Health Service, N. I. H.). Amino acids are numbered according to the Kabat system. The DNA sequence encoding the (Gly―Ser)₃ linker (45 bp) is identical to that of the linker-primer DNA provided with the Pharmacia scFv cloning system (Pharmacia Biotech), in which some codons differ from those originally published for this linker (Huston, J. S., et al. 1988. Protein engineering of antibody binding sites: Recovery of specific activity in an antigen-digoxin single-chain Fv analogue produced in *Escherichia coli. Proc. Natl. Acad. Sci. USA* 85: 5879-5883; Huston, J. S., et al. 1995. Single-chain Fv design and production by protein folding. In: *Antibody Engineering (Second Edition)*, eds. CAK Borrebaeck, pp. 185-227. New York: Oxford University Press). The V_{L} sequence includes the cysteines at positions 23 and 88 which form an intrachain disulfide bond and are probably essential for proper folding. It has been reported that Pro at positions 40 and 41 and Ser or Ala at positions 60-64 in the V_{H} domain of a recombinant antibody may affect host cell growth and promote antibody misfolding and leakage out of the periplasmic space (Byrne et al., 1996; Knappik and Plückthun, 1995). The V_{H} FR2 domain of p3A5.1c7 has Phe and Pro at positions 40 and 41, respectively, as do 97% of the documented mouse V_{H} sequences (Kabat et al., 1991). There is a Ser at residue 63 in complementarity determining region (CDR) H2, but residues 61-64 are homologous to 96% of mouse V_{H} sequences. Positively charged amino acids within the first 20-30 residues of the leader sequence have been reported to hinder transport of expressed proteins to the periplasm (Anderson, H. and von Heijne, G. 1991. A 30-residue long "export initiation domain" adjacent to the signal sequence is critical for protein translocation across the inner membrane of Escherichia coli. *Proc. Natl. Acad. Sci. USA* 88: 9751-9754; Ayala, M., et al. 1995. Variable region sequence modulates periplasmic export of a single-chain Fv antibody fragment in *Escherichia coli. BioTechniques* 18: 832-842). There is only one positively charged residue, Lys, at position 3 in V_{H} FR1. Thus p3A5.1c7 does not have sequence features known to reduce expression of functional scFv.

Because the first few N-terminal amino acids in antibody V_{H} and V_{L} domains are generally different, degenerate 5' primers are used to amplify the sequences by PCR. This can introduce amino acid substitutions and/or insertions that may affect affinity or other properties. To see whether this occurred, the N-terminal 15 amino acids of the parent MAb 3A5 H and L chains were determined (Table 4), and compared to those of the cloned scFv. The results are tabulated in Table 7 (Figure 18). Three of the first 6 residues of the V_{H} sequence and two of the first four in the V_{L} of the scFv were different, but the rest of the sequences were identical. The difference in affinity between scFv and MAb 3A5 may be caused by these amino acid substitutions.

### III. Applications

MAbs in accordance with this invention, including the stereospecific MAb 3A5 and its scFv and related derivatives, may be incorporated into virtually any immunoassay and immunosensor format and many immunoaffinity matrices. These would be useful and cost-effective in applications that include, but are not limited to, residue analysis on consumable crops, environmental fate, transport, and metabolism studies, quality control and registration of herbicide formulations, identification of violative uses, hazard assessment such as monitoring of worker exposure, spills, wind drift, and leaching into groundwater.

*Immunoaffinity chromatography with the MAbs.* Affinity columns prepared with MAb 3A2 were shown to be efficient and reproducible for recovery of imazethapyr residues. Such columns may also be prepared with MAbs 3A5 and its recombinant scFv derivative for stereospecific separations. Affinity matrices with the MAbs were prepared by the procedure summarized below, which is a standard method for persons familiar with the technique. To facilitate immobilization of the scFv, sequences coding for affinity tag peptides were inserted into the vector by site-directed mutagenesis.

*Example: Preparation of affinity column with MAb.* A saturating amount of monoclonal IgG is adsorbed onto a 5 mL Protein G cartridge (Hi-Trap Protein G, Pharmacia, Piscataway, NJ). The IgG is ten covalently linked to the Protein G with dimethylpimelimidate according to the method of Schneider, C., et al. 1982. A one-step purification of membrane proteins using a high efficiency immunomatrix. *J. Biol. Chem* 257: 10766-10769. Briefly, ascites solution may be diluted about 10 fold with about 20mM sodium phosphate buffer about pH 7.0 (PB) and applied onto a HiTrap G column via a peristaltic pump at flow rate of about 0.3 mL/min. After the entire sample has been applied, the column may be exhaustively washed with PB to remove non-specifically bound protein. The bound antibody may then be eluted with 0.1 M glycine buffer pH 2.5. The glycine eluant may be neutralized with 1 M Tris-HCl buffer pH 9.0 and dialyzed against PB. The activity of the purified antibody may be confirmed by enzyme immunoassay (1) and the protein concentration was determined by absorption at 280 nm.

To prepare an antibody column, a solution of purified antibody equivalent to about 3.5 mg of protein may be applied onto a HiTrap G 1 mL column. The column may be equilibrated with about 20mM ethanol amine about pH 8.2 followed with about 15 mL of about 30 mM dimethyl pimelimidate about pH 8.2 to cross-link the antibody. The column may then be washed with PB and the imazethapyr binding capacity of the column determined by using radio labeled imazethapyr following procedures well known in the art.

In a preferred embodiment of the present invention, a stereospecific monoclonal antibody having affinity for the *S*-isomer of an imidazolinone may be incorporated into an affinity column and used to separate a diastereomeric mixture of an imidazolinone. A sample of the diastereomeric imidazolinone mixture may be applied, for example, with a peristaltic pump at a flow rate between about 0.3 to 0.5 mL per minute, in a phosphate buffer at about neutral pH to the affinity column as described above. The non-binding fraction of the sample emerging from the column will contain the *R*-isomer of the imidazolinone. The biologically active *R*-isomer sample may be collected and, for example, used in herbicide formulations. The *S*-isomer bound fraction of the sample may then be eluted, for example with 30% methanol, as described above, and collected separately.

*Addition of Affinity Tags to p3A5.1c7*. Because scFvs lack constant domains and variants may have different chemical properties than the wild-type antibody, it is necessary to add an epitope or other biospecific affinity tag to facilitate purification, assay formatting, and preparation of affinity columns. The pCANTAB 5E vector in which the scFv was cloned included a 13-amino acid "E-tag" sequence that is expressed after the C-terminus of the V_{L} domain. The E-tag is recognized by a commercially available MAb. Very low yields of active p3A5.1c7 were recovered in attempts to affinity purify it on an anti-E-tag column. Two other affinity tags were introduced into the scFv by *in vitro* mutagenesis, and their effects on the purity, yield, and activity after affinity purification, and on activity and stability of the scFv in direct and indirect EIAs were investigated. Size and purity of the products were assessed by SDS-PAGE under disulfide bond reducing conditions, and western blot analysis.

The "mini-FLAG" pentapeptide (Asp-Tyr-Lys-Asp-Glu), the shortest tag sequence for which high-affinity antibodies are commercially available, is best recognized when it is at the N-terminus of the protein to be purified (Ge, L., et al. 1995. Expressing antibodies in *Escherichia coli.* In: *Antibody Engineering (2nd edition)*, ed. CAK Borrebaeck, pp. 229-266. New York: Oxford University Press.; Knappik, A. and Plückthun, A. 1994. An improved affinity tag based on the FLAG peptide for the detection and purification of recombinant antibody fragments. *BioTechniques* 17: 754-761). An insertion coding for Asp-Tyr-Lys-Asp was made adjacent to the site where the leader sequence is cleaved. To maximize recognition of the mini-FLAG, the N-terminal glutamine of the V_{H} (nucleotides 2347-2349) was changed to glutamic acid (CAA --> GAA). The mutagenesis was done as described above in Methods, using an oligonucleotide primer that included 11 extra amino acids and restored the *Sfi I* restriction site at the 5 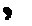 end of p3A5.1c7. The p3A5.1c7-FLAG was purified from periplasmic lysates of induced *E. coli* HB2151 using M1 MAb affinity gel. Active scFv was purified by loading the column in the presence of Ca⁺² ion, and eluting with a pH 7 buffer containing EDTA. The scFv could also be eluted at pH 3.5. Western blots showed that the recovered scFv was nearly homogeneous, but the yields were low. Purified p3A5.1c7-FLAG and the original p3A5.1c7 bound **16**-BSA identically.

The decapeptide (Ser-Ala-Trp-Arg-His-Pro-Gln-Phe-Gly-Gly) binds to streptavidin (Schmidt, T. G. M. and Skerra, A., 1993). The random peptide library-assisted engineering of a C-terminal affinity peptide, useful for the detection and purification of a functional Ig Fv fragment. *Protein Eng.* 6: 109-122. ScFvs with this "strep-tag" can be affinity purified by binding to streptavidin-agarose and elution with diaminobiotin in a gentle physiological buffer. The strep-tag can be detected in EIAs and western blots using streptavidin-enzyme conjugates, and it can be used to immobilize the scFv on biosensors. A form of p3A5.1c7 with the strep-tag was constructed followed by a stop codon inserted between the C terminus of the V_{L} sequence and the E-tag sequence in the pCANTAB 5E vector. The stop codon prevented translation of the E-tag, which blocked the binding activity of the strep-tag. This p3A5.1c7-strep construct efficiently bound streptavidin-AP in indirect EIAs and western blots, and it could be affinity purified to near homogeneity with roughly 71% yield. The protein from die most active eluted fractions resolved in a single band with an apparent size of 30.6 kDa, the calculated molecular weight for the scFv. Affinity purified p3A5.1c7-strep could be used at a 60-fold dilution in competition EIAs. The I₅₀ values for imazethapyr (PURSUIT) were 60 and 30 ppb, respectively, and the I₅₀ values for CADRE were 72 and 62 ppb, respectively.

Of course, antibodies in accordance with the present invention may also be used in other types of assay formats and analytical techniques including the following (references to various implementations that may be adapted to use antibodies in accordance with the present invention are noted):
Microplates and coated tubes, spheres, etc. (Kurosawa, S., et al. 1990. Latex piezoelectric immunoassay - detection of agglutination of antibody-bearing latex using a piezoelectric quartz crystal. *Chemical and Pharmaceutical Bulletin* 38: 1117-1120; Nilsson, K. G. I. 1989. Preparation of nanoparticles conjugated with enzyme and antibody, and their use in heterogeneous enzyme immunoassays. *J. Immunol. Methods* 122: 273-277; California Environmental Protection Agency. 1995. EnviroGard™ Immunoassay for PCBs in Soil (Millipore Corp.): Evaluation Report and Certification Statement; Ferguson, B. S., et al.: 1995. The detection and quantitation of imazapyr (Arsenal) in soil by enzyme immunoassay. In: Proceedings of Abstracts of Papers, 210th American Chemical Society National Meeting, August 20-24, 1995, Chicago, Illinois, USA,, American Chemical Society, v.210, n.1-2, (1995): AGRO pp. AGRO 7.);
Dipsticks and cards (Rittenburg, J. H., et al. 1991. Rapid On-Site Immunoassay Systems. In: *Immunoassays for Trace Chemical Analysis,* eds. M Vanderlaan, L Stanker, B Watkins, D Roberts, pp. 28-39. Washington, D.C.: Amer. Chem. Soc.; Collins, W. P. 1985. *Alternative Immunoassays,* Chichester: J. Wiley & Sons.; Inbar, S., et al. 1990. Dry chemistry thin film immunoassay. *Ann. Biol. Clin. Paris* 48: 385-390.; Grenner, G., et al. 1989. Multilayer fluorescent immunoassay technique. *Clin. Chem.* 35: 1865-1868);
Radioimmunoassay (Fránek, M., et al. 1992. Development of a microcolumn radioimmunoassay for screening of polychlorinated biphenyls in milk and animal fats. *J. Agric. Food Chem.* 40: 1559-1565);
Liposome immunoassay (Lee, M. and Durst, R. A. 1996. Determination of imazethapyr using capillary column flow injection liposome immunoanalysis. *J. Agric. Food Chem.* 44: 4032-4036; Locasio-Brown, L., et al. 1990. Liposome flow-injection immunoassay ― implications for sensitivity, dynamic range, and antibody regeneration. *Anal. Chem.* 6: 2587-2593; Monroe, D. 1989. Liposome immunoassay: A technology utilizing liposomes for the quantitation of analytes and immune reactions. Immunochemica (pub. by Zymed Laboratories, South San Francisco, CA): pp. 1-4, vol. 3 no. 1; Monroe, D. 1986. Liposome Immunoassay. In: *Immunoassay Technology*, eds. SB Pal, pp. 167-187. Berlin: Walter de Gruyter & Co.; Nakamura, T., et al. 1989. A liposome immunoassay based on a chemiluminescence reaction. *Chemical and Pharmaceutical Bulletin* 37: 1629-1631);
Assays using chemiluminescent or europium chelate substrates (Arfeyev, A. A., et al. 1990. Flow-injection immunoassay of haptens with enhanced chemiluminescence detection. *Analyt. Chim. Acta* 237: 285-289; Morton, R. C. and Diamandis, E. P. 1990. Streptavidin-based macromolecular complex labeled with a europium chelator, suitable for time-resolved fluorescence immunoassay applications. *Anal. Chem.* 62: 1841-1845);
Fiber-optic sensor with fluorescence detection (Walt, D. R., et al. 1995. Self-regenerating fiber-optic sensors. In: *Immunoanalysis of Agrochemicals: Emerging Technologies*, eds. J Nelson, AE Karu, R Wong, pp. 186-196. Washington D.C.: American Chemical Society; Anis, N. A. and Eldefrawi, M. E. 1993. Reusable fiber optic immunosensor for rapid detection of imazethapyr herbicide. *J. Agric. Food Chem.* 41: 843-848; Eldefrawi, M. E., et al. 1995. Fiber-optic immunosensors for detection of pesticides. In: *Immunoanalysis of Agrochemicals: Emerging Technologies*, eds. J Nelson, AE Karu, R Wong, pp. 197-209. Washington D.C.: American Chemical Society; Lovelt, R. A. July 1998. Australian researchers develop "nanosensor". Inside Laboratory Management (AOAC International monthly) : pp. 12-14; Vo-Dinh, T., et al. 1987. Antibody-based fiberoptics biosensor for the carcinogen benzo[*a*]pyrene. *Appl. Spectros.* 41: 735-738; Wadkins, R. M., et al. 1995. Calibration of biosensor response using simultaneous evanescent wave excitation of cyanine-labeled capture antibodies and antigens. *Anal. Biochem.* 232: 73-78);
Capacitive, resistive, and amperometric immunosensors (Milagres, B. G., et al. 1996. Immobilized ferrocene and glucose oxidase on titanium(IV) oxide grafted onto a silica gel surface and its application as an amperometric glucose biosensor. *Electroanalysis* 8: 489-493; Parce, J. W., et al. 1989. High sensitivity silicon biosensors designed for continuous environmental monitoring. *(Molecular Devices, Inc., Menlo Park, CA) Presented at Immunoassay Symposium, International Chemical Congress of Pacific Basin Societies, Honolulu, HI, Dec. 17-22*; Schuhmann, W. 1993. Non-leaking amperometric biosensors based on high-molecular ferrocene derivatives. *Biosensors & Bioelectronics* 8: 191-196; Monroe, D. 1990. Amperometric Immunoassay. *CRC Critical Reviews in Clinical Laboratory Sciences* 28: 1-18);
Fluorescence polarization immunoassays;
Immunoelectrodes (Foulds, N. E., et al. 1990. Immunoelectrodes. In: *Biosensors: A Practical Approach*, eds. AEG Cass, pp. 97-124. Oxford: IRL Press; ion-channel immunosensors (Cornell, B. A., et al. 1997. A biosensor that uses ion-channel switches. *Nature* 387: 580-583; Cornell, B. A., et al. 1997. A synthetic gated ion channel within a synthetic tethered membrane. (41st Annual Meeting of the Biophysical Society, New Orleans, Louisiana, USA. *Biophys. J.* 72 (Part 2): A397; Lovett, R. A. 1998. Australian researchers develop "nanosensor". Inside Laboratory Management (AOAC International monthly) : pp. 12-14, vol. July);
Immuno-PCR (immunoassay with PCR detection) (Sano, T., et al. 1992. Immuno-PCR: very sensitive antigen detection by means of specific antibody-DNA conjugates. *Science* 258: 120-122);
Affinity-based methods (Valkirs, G. E. and Barton, R 1985. Immunoconcentration™: a new format for solid-phase immunoassays. *Clin. Chem.* 31: 1427; Candlish, A. A. G., et al. 1991. Immunological Methods for Aflatoxins: Collaborative Study of Immunoaffinity Column Chromatography Method. In: *Food Safety and Quality Assurance: Applications of Immunoassay Systems,* eds. CJ Smith, MRA Morgan, Windermere, England: Elsevier Co.; Degan, P., et al. 1991. Immunoaffinity isolation of urinary 8-hydroxy-2 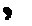 deoxyguanosine and 8-hydroxyguanine and quantitation of 8-hydroxy-2 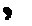 deoxyguanosine in DNA by polyclonal antibodies. *Carcinogenesis* 12: 865-871; Groopman, J. and Zarba, A. 1991. Immunoaffinity-Based Monitoring of Human Exposure to Aflatoxins in China and Gambia. In: *Immunoassays for Trace Chemical Analysis*, eds. M Vanderlaan, L, Stanker, B Watkins, D Roberts, pp. 207-214. Washington, D.C.: Amer. Chem. Soc.; Phillips, T. 1989. High-performance immunoaffinity chromatography. In: Advances in Chromatography, edited by J Giddings, E Gruschka, P Brown, pp. 134-149. New York: Marcel Dekker; Wong, R. B., et al. 1995. Immunoaffinity chromatography applications in pesticide metabolism and residue analyses. In: *Immunoanalysis of Agrochemicals: Emerging Technologies,* eds. J Nelson, AE Karu, R Wong, pp. 235-247. Washington D.C.: American Chemical Society; Zuehlke, J., et al. 1995. Sol-gel glass as a new support matrix in immunoaffinity chromatography. *Fresenius' Journal of Analytical Chemistry* 352: 654-659); and,
Multi-analyte methods (Ekins, R., et al. 1989. Development of microspot multianalyte ratiometric immunoassay using dual fluorescent-labeled antibodies. *Analyt. Chim. Acta* 227: 73-96.; Ekins, R., et al. 1990. Multispot, multi-analyte immunoassay. *Ann. de Biol. Clinique* 48: 655-666; Ekins, R. P. and Chu, F. W. 1995. Miniaturized microspot multi-analyte immunoassay systems. In: *Immunoanalysis of Agrochemicals: Emerging Technologies*, eds. J Nelson, AE Karu, R Wong, pp. 153-174. Washington D.C.: American Chemical Society (Symposium No. 586); Ekins, R. P., et al. 1990. Multi-analyte immunoassay ― the immunological compact disk of the future. *Journal of Clinical Immunoassay* 13: 169-181; Cheung, P. Y. K., et al. 1993. Harnessing immunochemical cross-reactivity: use of pattern recognition to classify molecular analogs. *Analyt. Chim. Acta* 282: 181-192; Karu, A. E., et al. 1994. Use of multivariate statistical methods to identify immunochemical cross-reactants. *Food & Agric. Immunol.* 6: 371-384).

Also, stereospecific monoclonal antibodies might be expressed in plants, where they might compete for imidazolinone binding with acetohydroxyacid synthase, which is the natural target. This might make it possible to modulate the uptake and bioavailability of the herbicide and engineer imidazolinone-resistant plants.

### IV. Conclusion

The present invention provides monoclonal antibodies and derivatives which have chiral specificity for imidazolinones. A series of haptens resembling the herbicide imazethapyr (PURSUIT®) was synthesized and used to derive a panel of monoclonal antibodies (MAbs) that detect imazethapyr, imazaquin (SCEPTER®), imazapic (CADRE®), and imazamox (RAPTOR®) in the 3-30 ng/mL (parts per billion) range, and imazapyr (ARSENAL®) and imazamethabenz-methyl (ASSERT®) in the 300-500 ppb range. MAbs specific for the *S* isomers of the imidazolinone herbicides were identified from the panel. Some of the MAbs are stable in up to 15% methanol and 5% acetonitrile. Substrates coated with conjugates for indirect EIA may be stored frozen, and selectivity of some MAbs is different on different coating conjugates. The MAbs are suitable for imidazolinone herbicide analysis and immunoaffinity residue recovery and stereospecific separations.

The haptens and MAbs described in this disclosure should be usable in any immunoassay format ― field-portable cards, dipsticks, sensors, etc. They could be used in methods such as immuno-PCR to achieve sensitive to very few imidazolinone molecules. Using recombinant DNA methods the genes can be cloned from these MAbs, or synthetic combinatorial antibody libraries can be subjected to selection on the hapten conjugates described herein. This could facilitate the derivation or engineering of antibodies with new specificities. It also would enable cloning of the antibody genes into selected crop plants, where expression could confer the ability to resist or take up imidazolinones. Additionally, it may be possible to engineer antibodies that catalyze the breakdown of imidazolinones.

A preferred embodiment of this aspect of the present invention is designated MAb 3A5.1. MAb 3A5.1 is an IgG₂ₐk immunoglobulin. It binds four of the five most commonly used imidazolinone herbicides, imazethapyr (PURSUIT®), imazamethabenz-methyl (ASSERT®), imazapic (CADRE), and imazamox (RAPTOR ®), in direct and indirect EIAs, with strong preference for the *S* chiral isomers. This chiral specificity may be applied for analysis and stereospecific separation of formulations and residues.

In addition to the elucidation, identification and application of stereospecific monoclonal antibodies, the present invention provides a single-chain Fv antibody (scFv), specific for imazethapyr and related imidazolinone herbicides. A preferred embodiment of this scFv, designated p3A5.1 c7, was prepared by cloning the variable heavy (V_{H}) and variable light (V_{L}) domains from a hybridoma (3A5.1), which produces MAb with chiral specificity for the *S*-isomer of imidazolinones. The scFv molecules were much smaller (26-27 kDa) than intact IgG antibodies (150 kDa). A preferred embodiment of this scFv aspect of the present invention, selected in a commercial phage display system, has about the same specificity as a preferred embodiment of the monoclonal antibody (MAb) aspect for *S* isomers of imidazolinones. The scFv may be less susceptible to denaturation by methanol and acetonitrile than the MAb, but the detection limit for free imidazolinones in indirect enzyme immunoassays (EIAs) with the scFv may also be less sensitive.

In addition, stereospecific monoclonal antibodies in accordance with the present invention might be expressed in plants, where they might compete for imidazolinone binding with acetohydroxyacid synthase, which is the natural target. This might make it possible to modulate the uptake and bioavailability of the herbicide.

All references cited in this application are incorporated by reference herein for all purposes

Although the foregoing invention has been described in some detail for purposes of clarity of understanding, it will be apparent that certain changes and modifications may be practiced within the scope of the appended claims. It should be noted that there may be alternative ways of implementing both the processes and compositions of the present invention. Accordingly, the present embodiments are to be considered as illustrative and not restrictive, and the invention is not to be limited to the details given herein, but may be modified within the scope and equivalents of die appended claims.

## Claims

1. A monoclonal antibody for an imidazolinone herbicide, said monoclonal antibody exhibiting chiral specificity for an enantiomer of said imidazolinone herbicide.

2. The monoclonal antibody of claim 1, wherein said monoclonal antibody has *S*-isomer chiral specificity.

3. The monoclonal antibody of claim 1, wherein said imidazolinone herbicide is selected from the group consisting of imazethapyr, imazaquin, imazapyr, and imazamox.

4. The monoclonal antibody of claim 2, wherein said monoclonal antibody comprises an IgG2a heavy chain having an N-terminal amino acid sequence of SEQ. ID No. 1, and a kappa light chain having an N-terminal amino acid sequence of SEQ. ID No. 2.

5. The monoclonal antibody of claim 2, wherein said monoclonal antibody has affinity for the S-isomer at least 10 times greater than for the R-isomer.

6. An immunoaffinity assay for chiral isomers of an imidazolinone herbicide, comprising:
contacting a sample comprising one or more imidazolinones with a monoclonal antibody for an imidazolinone herbicide, said monoclonal antibody exhibiting chiral specificity for an enantiomer of said imidazolinone herbicide.

7. The method of claim 6, further comprising determining the presence of an enantiomer of the imidazolinone herbicide bound to said monoclonal antibody.

8. The method of claim 7, further comprising quantifying the presence of an enantiomer of the imidazolinone herbicide bound to said monoclonal antibody.

9. The method of claim 6, wherein said enantiomer is an S-isomer of said imidazolinone herbicide.

10. An immunoaffinity separation method for chiral isomers of an imidazolinone herbicide, comprising:
contacting a sample comprising one or more imidazolinones with a monoclonal antibody for an imidazolinone herbieide immobilized on a solid material, said monoclonal antibody exhibiting chiral specificity for an enantiomer of said imidazolinone herbicide.

11. The method of claim 10, wherein said separation method uses chromatography.

12. The method of claim 10, wherein said enantiomer is an S-isomer of said imidazolinone herbicide.

13. An apparatus for conducting an immunoaffinity assay for chiral isomers of an imidazolinone herbicide, comprising:
a monoclonal antibody for an imidazolinone herbicide covalently immobilized on a solid material, said monoclonal antibody exhibiting chiral specificity for an enantiomer of said imidazolinone herbicide.

14. The apparatus of claim 13, wherein said apparatus is an immunoaffinity dipstick.

15. The apparatus of claim 13, wherein said enantiomer is an S-isomer of said imidazolinone herbicide.

16. An immunoaffinity separation column for chiral isomers of an imidazolinone herbicide, comprising:
a monoclonal antibody for an imidazolinone herbicide immobilized on a solid column-packing material, said monoclonal antibody exhibiting chiral specificity for an enantiomer of said imidazolinone herbicide.

17. The column of claim 16, wherein said immunoaffinity separation column is a HiTrap G 1 mL column.

18. The column of claim 16, wherein said enantiomer is an S-isomer of said imidazolinone herbicide.

19. A recombinant single-chain Fv antibody for an imidazolinone herbicide, said antibody exhibiting chiral specificity for an enantiomer of said imidazolinone herbicide.

20. The antibody of claim 19, wherein said antibody has *S*-isomer chiral specificity.

21. The antibody of claim 19, wherein said imidazolinone herbicide is selected from the group consisting of imazethapyr and imazaquin.

22. The antibody of claim 19, wherein said antibody comprises a heavy chain variable domain and a light chain variable domain cloned from a cell line for a monoclonal antibody having chiral specificity for said enantiomer of said imidazolinone herbicide.

23. The antibody of claim 20, wherein said antibody comprises a heavy chain variable domain cloned from a cell line for a monoclonal antibody having chiral specificity for said enantiomer of said imidazolinone herbicide and having an N-terminal amino acid sequence of SEQ. ID No. 5; and a light chain variable domain cloned from a cell line for a monoclonal antibody having chiral specificity for said enantiomer of said imidazolinone herbicide and having an N-terminal amino acid sequence of SEQ. ID No. 6.

24. An immunoaffinity assay for chiral isomers of an imidazolinone herbicide, comprising:
contacting a sample comprising one or more imidazolinones with a single-chain Fv antibody for an imidazolinone, said antibody exhibiting chiral specificity for an enantiomer of said imidazolinone herbicide.

25. The method of claim 24, further comprising determining the presence of an enantiomer of the imidazolinone herbicide bound to said antibody.

26. The method of claim 24, wherein said enantiomer is an S-isomer of said imidazolinone herbicide.

27. An immunoaffinity separation method for chiral isomers of an imidazolinone herbicide, comprising:
contacting a sample comprising one or more imidazolinones with a single-chain Fv antibody for an imidazolinone herbicide immobilized on a solid column-packing material, said antibody exhibiting chiral specificity for an enantiomer of said imidazolinone herbicide.

28. The method of claim 27, wherein said separation method uses chromatography. - -

29. The method of claim 27, wherein said enantiomer is an S-isomer of said imidazolinone herbicide.

30. An apparatus for conducting an immunoaffinity assay for chiral isomers of an imidazolinone herbicide, comprising:
a single-chain Fv antibody for an imidazolinone herbicide immobilized on a solid material, said antibody exhibiting chiral specificity for an enantiomer of said imidazolinone herbicide.

31. The apparatus of claim 30, further comprising a biospecific affinity tag bound to said antibody.

32. The apparatus of claim 30, wherein said enantiomer is an S-isomer of said imidazolinone herbicide.

33. The apparatus of claim 30, wherein said apparatus is an immunoaffinity dipstick.

34. An immunoaffinity separation column for chiral isomers of an imidazolinone herbicide, comprising:
a single-chain Fv antibody for an imidazolinone herbicide immobilized on a solid column-packing material, said antibody exhibiting chiral specificity for an enantiomer of said imidazolinone herbicide.

35. The column of claim 34, further comprising a biospecific affinity tag bound to said antibody.

36. The column of claim 34, wherein said immunoaffinity separation column is a HiTrap G 1 ml column.

37. The column of claim 34, wherein said enantiomer is an S-isomer of said imidazolinone herbicide.
